# EUROPEAN PATENT APPLICATION

(11) **EP 2 554 658 A1**
(43) Date of publication of application: **06.02.2013**
(21) Application number: 11765760.1
(22) Date of filing: 31.03.2011
(51) Int. Cl.: C12N 5/07, C12N 5/0735, C12N 5/0775, C12N 5/0786, C12N 5/0789, C12N 5/0797, C07K 14/525, C07K 14/765, C07K 14/78, C07K 14/79

(54) **METHOD FOR CULTURING CELLS IN SYSTEM CONTAINING LAMININ-5**

(30) Priority: 31.03.2010 JP 2010084153
(71) Applicant: Oriental Yeast Co., Ltd., Tokyo 174-8505 (JP)
(72) Inventor: YASUDA, Hisataka, IItabashi-ku, Tokyo 174-8505 (JP); YAMADA, Munehiro, Nagahama-shi Shiga 526-0804 (JP); TAKETANI, Yukiko, Nagahama-shi Shiga 526-0804 (JP); TOMIMORI, Yoshiya, Nagahama-shi Shiga 526-0804 (JP); MORI, Kaoru, Nagahama-shi Shiga 526-0804 (JP)
(74) Representative: Benedum, Ulrich Max
(86) International application number: PCT/JP2011/058265
(87) International publication number: WO 2011/125860

(57) **Abstract**

The present invention is directed to providing a method for culturing cells in a system containing laminin-5. The method of the present invention is characterized by a culture system containing a polypeptide selected from a group consisting of: a protein in blood other than extracellular matrix proteins, which is, serum, serum albumin, prealbumin, immunoglobulin, α-globulin, β-globulin, α1-antitrypsin (α1-AT), heptoglobin (Hp), α2-macroglobulin (α2-M), α-fetoprotein (AFP), transferrin, retinol-binding protein (RBP) or adiponectin; gelatin; a protein belonging to a tumor necrosis factor (TNF) family; and peptone.

SEQUENCE LISTING

<110> Oriental Yeast Co., Ltd.

<120> Method for culturing cells in a system comprising Laminin-5

<130> FA1312-11025

<150> JP 2010-84153
<151> 2010-03-31

<160> 14

<210> 1
<211> 5433
<212> DNA
<213> Human

<220>
<221> CDS
<222> (1)...(5139)

<300>
<301> Ryan,M.C., Tizard,R., VanDevanter,D.R. and Carter,W.G.
<302> Cloning of the LamA3 gene encoding the alpha 3 chain of
      the adhesive ligand epiligrin. Expression in wound repair
<303> JOURNAL   J. Biol. Chem.
<304> 269
<305> 36
<306> 22779-22787
<307> 1994

<400> 1
atg gga tgg ctg tgg atc ttt ggg gca gcc ctg ggg cag tgt ctg   45
Met Gly Trp Leu Trp Ile Phe Gly Ala Ala Leu Gly Gln Cys Leu
  1               5                  10                  15

ggc tac agt tca cag cag caa agg gtg cca ttt ctt cag cct ccc   90
Gly Tyr Ser Ser Gln Gln Gln Arg Val Pro Phe Leu Gln Pro Pro
                 20                  25                  30

ggt caa agt caa ctg caa gcg agt tat gtg gag ttt aga ccc agc  135
Gly Gln Ser Gln Leu Gln Ala Ser Tyr Val Glu Phe Arg Pro Ser
                 35                  40                  45

cag ggt tgt agc cct gga tac tat cgg gat cat aaa ggc ttg tat  180
Gln Gly Cys Ser Pro Gly Tyr Tyr Arg Asp His Lys Gly Leu Tyr
                 50                  55                  60

acc gga cgg tgt gtt ccc tgc aat tgc aac gga cat tca aat caa  225
Thr Gly Arg Cys Val Pro Cys Asn Cys Asn Gly His Ser Asn Gln
                 65                  70                  75

tgc cag gat ggc tca ggc ata tgt gtt aac tgt cag cac aac acc  270
Cys Gln Asp Gly Ser Gly Ile Cys Val Asn Cys Gln His Asn Thr
                 80                  85                  90

gcg gga gag cac tgt gaa cgc tgc cag gag ggc tac tat ggc aac  315
Ala Gly Glu His Cys Glu Arg Cys Gln Glu Gly Tyr Tyr Gly Asn
                 95                 100                 105

gcc gtc cac gga tcc tgc agg gcc tgc cca tgt cct cac act aac  360
Ala Val His Gly Ser Cys Arg Ala Cys Pro Cys Pro His Thr Asn
                110                 115                 120

agc ttt gcc act ggc tgt gtg gtg aat ggg gga gac gtg cgg tgc  405
Ser Phe Ala Thr Gly Cys Val Val Asn Gly Gly Asp Val Arg Cys
                125                 130                 135

tcc tgc aaa gct ggg tac aca gga aca cag tgt gaa agg tgt gca  450
Ser Cys Lys Ala Gly Tyr Thr Gly Thr Gln Cys Glu Arg Cys Ala
                140                 145                 150

ccg gga tat ttc ggg aat ccc cag aaa ttc gga ggt agc tgc caa  495
Pro Gly Tyr Phe Gly Asn Pro Gln Lys Phe Gly Gly Ser Cys Gln
                155                 160                 165

cca tgc agt tgt aac agc aat ggc cag ctg ggc agc tgt cat ccc 540
Pro Cys Ser Cys Asn Ser Asn Gly Gln Leu Gly Ser Cys His Pro
                170                 175                 180

ctg act gga gac tgc ata aac caa gaa ccc aaa gat agc agc cct 585
Leu Thr Gly Asp Cys Ile Asn Gln Glu Pro Lys Asp Ser Ser Pro
                185                 190                 195

gca gaa gaa tgt gat gat tgc gac agc tgt gtg atg acc ctc ctg 630
Ala Glu Glu Cys Asp Asp Cys Asp Ser Cys Val Met Thr Leu Leu
                200                 205                 210

aac gac ctg gcc acc atg ggc gag cag ctc cgc ctg gtc aag tct 675
Asn Asp Leu Ala Thr Met Gly Glu Gln Leu Arg Leu Val Lys Ser
                215                 220                 225

cag ctg cag ggc ctg agt gcc agc gca ggg ctt ctg gag cag atg 720
Gln Leu Gln Gly Leu Ser Ala Ser Ala Gly Leu Leu Glu Gln Met
                230                 235                 240

agg cac atg gag acc cag gcc aag gac ctg agg aat cag ttg ctc 765
Arg His Met Glu Thr Gln Ala Lys Asp Leu Arg Asn Gln Leu Leu
                245                 250                 255

aac tac cgt tct gcc att tca aat cat gga tca aaa ata gaa ggc 810
Asn Tyr Arg Ser Ala Ile Ser Asn His Gly Ser Lys Ile Glu Gly
                260                 265                 270

ctg gaa aga gaa ctg act gat ttg aat caa gaa ttt gag act ttg 855
Leu Glu Arg Glu Leu Thr Asp Leu Asn Gln Glu Phe Glu Thr Leu
                275                 280                 285

caa gaa aag gct caa gta aat tcc aga aaa gca caa aca tta aac 900
Gln Glu Lys Ala Gln Val Asn Ser Arg Lys Ala Gln Thr Leu Asn
                290                 295                 300

aac aat gtt aat cgg gca aca caa agc gca aaa gaa ctg gat gtg 945
Asn Asn Val Asn Arg Ala Thr Gln Ser Ala Lys Glu Leu Asp Val
                305                 310                 315

aag att aaa aat gtc atc cgg aat gtg cac att ctt tta aag cag 990
Lys Ile Lys Asn Val Ile Arg Asn Val His Ile Leu Leu Lys Gln
                320                 325                 330

atc tct ggg aca gat gga gag gga aac aac gtg cct tca ggt gac 1035
Ile Ser Gly Thr Asp Gly Glu Gly Asn Asn Val Pro Ser Gly Asp
                335                 340                 345

ttt tcc aga gag tgg gct gaa gcc cag cgc atg atg agg gaa ctg 1080
Phe Ser Arg Glu Trp Ala Glu Ala Gln Arg Met Met Arg Glu Leu
                350                 355                 360

cgg aac agg aac ttt gga aag cac ctc aga gaa gca gaa gct gat 1125
Arg Asn Arg Asn Phe Gly Lys His Leu Arg Glu Ala Glu Ala Asp
                365                 370                 375

aaa agg gag tcg cag ctc ttg ctg aac cgg ata agg acc tgg cag 1170
Lys Arg Glu Ser Gln Leu Leu Leu Asn Arg Ile Arg Thr Trp Gln
                380                 385                 390

aaa acc cac cag ggg gag aac aat ggg ctt gct aac agt atc cgg 1215
Lys Thr His Gln Gly Glu Asn Asn Gly Leu Ala Asn Ser Ile Arg
                395                 400                 405

gat tct tta aat gaa tac gaa gcc aaa ctc agt gac ctt cgt gct 1260
Asp Ser Leu Asn Glu Tyr Glu Ala Lys Leu Ser Asp Leu Arg Ala
                410                 415                 420

cgg ctg cag gag gca gct gcc caa gcc aag cag gca aat ggc ttg 1305
Arg Leu Gln Glu Ala Ala Ala Gln Ala Lys Gln Ala Asn Gly Leu
                425                 430                 435

aac caa gaa aac gag aga gct ttg gga gcc att cag aga caa gtg 1350
Asn Gln Glu Asn Glu Arg Ala Leu Gly Ala Ile Gln Arg Gln Val
                440                 445                 450

aaa gaa ata aat tcc ctg cag agt gat ttc acc aag tat cta acc 1395
Lys Glu Ile Asn Ser Leu Gln Ser Asp Phe Thr Lys Tyr Leu Thr
                455                 460                 465

act gca gac tca tct ttg ttg caa acc aac att gcg ctg cag ctg 1440
Thr Ala Asp Ser Ser Leu Leu Gln Thr Asn Ile Ala Leu Gln Leu
                470                 475                 480

atg gag aaa agc cag aag gaa tat gaa aaa tta gct gcc agt tta 1485
Met Glu Lys Ser Gln Lys Glu Tyr Glu Lys Leu Ala Ala Ser Leu
                485                 490                 495

aat gaa gca aga caa gaa cta agt gac aaa gta aga gaa ctt tcc 1530
Asn Glu Ala Arg Gln Glu Leu Ser Asp Lys Val Arg Glu Leu Ser
                500                 505                 510

aga tct gct ggc aaa aca tcc ctt gtg gag gag gca gaa aag cac 1575
Arg Ser Ala Gly Lys Thr Ser Leu Val Glu Glu Ala Glu Lys His
                515                 520                 525

gcg cgg tcc tta caa gag ctg gca aag cag ctg gaa gag atc aag 1620
Ala Arg Ser Leu Gln Glu Leu Ala Lys Gln Leu Glu Glu Ile Lys
                530                 535                 540

aga aac gcc agc ggg gat gag ctg gtg cgc tgt gct gtg gat gcc 1665
Arg Asn Ala Ser Gly Asp Glu Leu Val Arg Cys Ala Val Asp Ala
                545                 550                 555

gcc acc gcc tac gag aac atc ctc aat gcc atc aaa gcg gcc gag 1710
Ala Thr Ala Tyr Glu Asn Ile Leu Asn Ala Ile Lys Ala Ala Glu
                560                 565                 570

gac gca gcc aac agg gct gcc agt gca tct gaa tct gcc ctc cag 1755
Asp Ala Ala Asn Arg Ala Ala Ser Ala Ser Glu Ser Ala Leu Gln
                575                 580                 585

aca gtg ata aag gaa gat ctg cca aga aaa gct aaa acc ctg agt 1800
Thr Val Ile Lys Glu Asp Leu Pro Arg Lys Ala Lys Thr Leu Ser
                590                 595                 600

tcc aac agt gat aaa ctg tta aat gaa gcc aag atg aca caa aag 1845
Ser Asn Ser Asp Lys Leu Leu Asn Glu Ala Lys Met Thr Gln Lys
                605                 610                 615

aag cta aag caa gaa gtc agt cca gct ctc aac aac cta cag caa 1890
Lys Leu Lys Gln Glu Val Ser Pro Ala Leu Asn Asn Leu Gln Gln
                620                 625                 630

acc ctg aat att gtg aca gtt cag aaa gaa gtg ata gac acc aat 1935
Thr Leu Asn Ile Val Thr Val Gln Lys Glu Val Ile Asp Thr Asn
                635                 640                 645

ctc aca act ctc cga gat ggt ctt cat ggg ata cag aga ggt gat 1980
Leu Thr Thr Leu Arg Asp Gly Leu His Gly Ile Gln Arg Gly Asp
                650                 655                 660

att gat gct atg atc agt agt gca aag agc atg gtc aga aag gcc 2025
Ile Asp Ala Met Ile Ser Ser Ala Lys Ser Met Val Arg Lys Ala
                665                 670                 675

aac gac atc aca gat gag gtt ctg gat ggg ctc aac ccc atc cag 2070
Asn Asp Ile Thr Asp Glu Val Leu Asp Gly Leu Asn Pro Ile Gln
                680                 685                 690

aca gat gtg gaa aga att aag gac acc tat ggg agg aca cag aac 2115
Thr Asp Val Glu Arg Ile Lys Asp Thr Tyr Gly Arg Thr Gln Asn
                695                 700                 705

gaa gac ttc aaa aag gct ctg act gat gca gat aac tcg gtg aat 2160
Glu Asp Phe Lys Lys Ala Leu Thr Asp Ala Asp Asn Ser Val Asn
                710                 715                 720

aag tta acc aac aaa cta cct gat ctt tgg cgc aag att gaa agt 2205
Lys Leu Thr Asn Lys Leu Pro Asp Leu Trp Arg Lys Ile Glu Ser
                725                 730                 735

atc aac caa cag ctg ttg ccc ttg gga aac atc tct gac aac atg 2250
Ile Asn Gln Gln Leu Leu Pro Leu Gly Asn Ile Ser Asp Asn Met
                740                 745                 750

gac aga ata cga gaa cta att cag cag gcc aga gat gct gcc agt 2295
Asp Arg Ile Arg Glu Leu Ile Gln Gln Ala Arg Asp Ala Ala Ser
                755                 760                 765

aag gtt gct gtc ccc atg agg ttc aat ggt aaa tct gga gtc gaa 2340
Lys Val Ala Val Pro Met Arg Phe Asn Gly Lys Ser Gly Val Glu
                770                 775                 780

gtc cga ctg cca aat gac ctg gaa gat ttg aaa gga tat aca tct 2385
Val Arg Leu Pro Asn Asp Leu Glu Asp Leu Lys Gly Tyr Thr Ser
                785                 790                 795

ctg tcc ttg ttt ctc caa agg ccc aac tca aga gaa aat ggg ggt 2430
Leu Ser Leu Phe Leu Gln Arg Pro Asn Ser Arg Glu Asn Gly Gly
                800                 805                 810

act gag aat atg ttt gtg atg tac ctt gga aat aaa gat gcc tcc 2475
Thr Glu Asn Met Phe Val Met Tyr Leu Gly Asn Lys Asp Ala Ser
                815                 820                 825

cgg gac tac atc ggc atg gca gtt gtg gat ggc cag ctc acc tgt 2520
Arg Asp Tyr Ile Gly Met Ala Val Val Asp Gly Gln Leu Thr Cys
                830                 835                 840

gtc tac aac ctg ggg gac cgt gag gct gaa ctc caa gtg gac cag 2565
Val Tyr Asn Leu Gly Asp Arg Glu Ala Glu Leu Gln Val Asp Gln
                845                 850                 855

atc ttg acc aag agt gag act aag gag gca gtt atg gat cgg gtg 2610
Ile Leu Thr Lys Ser Glu Thr Lys Glu Ala Val Met Asp Arg Val
                860                 865                 870

aaa ttt cag aga att tat cag ttt gca agg ctt aat tac acc aaa 2655
Lys Phe Gln Arg Ile Tyr Gln Phe Ala Arg Leu Asn Tyr Thr Lys
                875                 880                 885

gga gcc aca tcc agt aaa cca gaa aca ccc gga gtc tat gac atg 2700
Gly Ala Thr Ser Ser Lys Pro Glu Thr Pro Gly Val Tyr Asp Met
                890                 895                 900

gat ggt aga aat agc aat aca ctc ctt aat ttg gat cct gaa aat 2745
Asp Gly Arg Asn Ser Asn Thr Leu Leu Asn Leu Asp Pro Glu Asn
                905                 910                 915

gtt gta ttt tat gtt gga ggt tac cca cct gat ttt aaa ctt ccc 2790
Val Val Phe Tyr Val Gly Gly Tyr Pro Pro Asp Phe Lys Leu Pro
                920                 925                 930

agt cga cta agt ttc cct cca tac aaa ggt tgt att gaa tta gat 2835
Ser Arg Leu Ser Phe Pro Pro Tyr Lys Gly Cys Ile Glu Leu Asp
                935                 940                 945

gac ctc aat gaa aat gtt ctg agc ttg tac aac ttc aaa aaa aca 2880
Asp Leu Asn Glu Asn Val Leu Ser Leu Tyr Asn Phe Lys Lys Thr
                950                 955                 960

ttc aat ctc aac aca act gaa gtg gag cct tgt aga agg agg aag 2925
Phe Asn Leu Asn Thr Thr Glu Val Glu Pro Cys Arg Arg Arg Lys
                965                 970                 975

gaa gag tca gac aaa aat tat ttt gaa ggt acg ggc tat gct cga 2970
Glu Glu Ser Asp Lys Asn Tyr Phe Glu Gly Thr Gly Tyr Ala Arg
                980                 985                 990

gtt cca act caa cca cat gct ccc atc cca acc ttt gga cag aca 3015
Val Pro Thr Gln Pro His Ala Pro Ile Pro Thr Phe Gly Gln Thr
                995                 1000                1005

att cag acc acc gtg gat aga ggc ttg ctg ttc ttt gca gaa aac 3060
Ile Gln Thr Thr Val Asp Arg Gly Leu Leu Phe Phe Ala Glu Asn
                1010                1015                1020

ggg gat cgc ttc ata tct cta aat ata gaa gat ggc aag ctc atg 3105
Gly Asp Arg Phe Ile Ser Leu Asn Ile Glu Asp Gly Lys Leu Met
                1025                1030                1035

gtg aga tac aaa ctg aat tca gag cta cca aaa gag aga gga gtt 3150
Val Arg Tyr Lys Leu Asn Ser Glu Leu Pro Lys Glu Arg Gly Val
                1040                1045                1050

gga gac gcc ata aac aac ggc aga gac cat tcg att cag atc aaa 3195
Gly Asp Ala Ile Asn Asn Gly Arg Asp His Ser Ile Gln Ile Lys
                1055                1060                1065

att gga aaa ctc caa aag cgt atg tgg ata aat gtg gac gtt caa 3240
Ile Gly Lys Leu Gln Lys Arg Met Trp Ile Asn Val Asp Val Gln
                1070                1075                1080

aac act ata att gat ggt gaa gta ttt gat ttc agc aca tat tat 3285
Asn Thr Ile Ile Asp Gly Glu Val Phe Asp Phe Ser Thr Tyr Tyr
                1085                1090                1095

ctg gga gga att cca att gca atc agg gaa aga ttt aac att tct 3330
Leu Gly Gly Ile Pro Ile Ala Ile Arg Glu Arg Phe Asn Ile Ser
                1100                1105                1110

acg cct gct ttc cga ggc tgc atg aaa aat ttg aag aaa acc agt 3375
Thr Pro Ala Phe Arg Gly Cys Met Lys Asn Leu Lys Lys Thr Ser
                1115                1120                1125

ggt gtc gtt aga ttg aat gat act gtg gga gta acc aaa aag tgc 3420
Gly Val Val Arg Leu Asn Asp Thr Val Gly Val Thr Lys Lys Cys
                1130                1135                1140

tcg gaa gac tgg aag ctt gtg cga tct gcc tca ttc tcc aga gga 3465
Ser Glu Asp Trp Lys Leu Val Arg Ser Ala Ser Phe Ser Arg Gly
                1145                1150                1155

gga caa ttg agt ttc act gat ttg ggc tta cca cct act gac cac 3510
Gly Gln Leu Ser Phe Thr Asp Leu Gly Leu Pro Pro Thr Asp His
                1160                1165                1170

ctc cag gcc tca ttt gga ttt cag acc ttt caa ccc agt ggc ata 3555
Leu Gln Ala Ser Phe Gly Phe Gln Thr Phe Gln Pro Ser Gly Ile
                1175                1180                1185

tta tta gat cat cag aca tgg aca agg aac ctg cag gtc act ctg 3600
Leu Leu Asp His Gln Thr Trp Thr Arg Asn Leu Gln Val Thr Leu
                1190                1195                1200

gaa gat ggt tac att gaa ttg agc acc agc gat agc ggc ggc cca 3645
Glu Asp Gly Tyr Ile Glu Leu Ser Thr Ser Asp Ser Gly Gly Pro
                1205                1210                1215

att ttt aaa tct cca cag acg tat atg gat ggt tta ctg cat tat 3690
Ile Phe Lys Ser Pro Gln Thr Tyr Met Asp Gly Leu Leu His Tyr
                1220                1225                1230

gta tct gta ata agc gac aac tct gga cta cgg ctt ctc atc gat 3735
Val Ser Val Ile Ser Asp Asn Ser Gly Leu Arg Leu Leu Ile Asp
                1235                1240                1245

gac cag ctt ctg aga aat agc aaa agg cta aaa cac att tca agt 3780
Asp Gln Leu Leu Arg Asn Ser Lys Arg Leu Lys His Ile Ser Ser
                1250                1255                1260

tcc cgg cag tct ctg cgt ctg ggc ggg agc aat ttt gag ggt tgt 3825
Ser Arg Gln Ser Leu Arg Leu Gly Gly Ser Asn Phe Glu Gly Cys
                1265                1270                1275

att agc aat gtt ttt gtc cag agg tta tca ctg agt cct gaa gtc 3870
Ile Ser Asn Val Phe Val Gln Arg Leu Ser Leu Ser Pro Glu Val
                1280                1285                1290

cta gat ttg acc agt aac tct ctc aag aga gat gtg tcc ctg gga 3915
Leu Asp Leu Thr Ser Asn Ser Leu Lys Arg Asp Val Ser Leu Gly
                1295                1300                1305

ggc tgc agt tta aac aaa cca cct ttt cta atg ttg ctt aaa ggt 3960
Gly Cys Ser Leu Asn Lys Pro Pro Phe Leu Met Leu Leu Lys Gly
                1310                1315                1320

tct acc agg ttt aac aag acc aag act ttt cgt atc aac cag ctg 4005
Ser Thr Arg Phe Asn Lys Thr Lys Thr Phe Arg Ile Asn Gln Leu
                1325                1330                1335

ttg cag gac aca cca gtg gcc tcc cca agg agc gtg aag gtg tgg 4050
Leu Gln Asp Thr Pro Val Ala Ser Pro Arg Ser Val Lys Val Trp
                1340                1345                1350

caa gat gct tgc tca cca ctt ccc aag acc cag gcc aat cat gga 4095
Gln Asp Ala Cys Ser Pro Leu Pro Lys Thr Gln Ala Asn His Gly
                1355                1360                1365
gcc ctc cag ttt ggg gac att ccc acc agc cac ttg cta ttc aag 4140
Ala Leu Gln Phe Gly Asp Ile Pro Thr Ser His Leu Leu Phe Lys
                1370                1375                1380

ctt cct cag gag ctg ctg aaa ccc agg tca cag ttt gct gtg gac 4185
Leu Pro Gln Glu Leu Leu Lys Pro Arg Ser Gln Phe Ala Val Asp
                1385                1390                1395

atg cag aca aca tcc tcc aga gga ctg gtg ttt cac acg ggc act 4230
Met Gln Thr Thr Ser Ser Arg Gly Leu Val Phe His Thr Gly Thr
                1400                1405                1410

aag aac tcc ttt atg gct ctt tat ctt tca aaa gga cgt ctg gtc 4275
Lys Asn Ser Phe Met Ala Leu Tyr Leu Ser Lys Gly Arg Leu Val
                1415                1420                1425

ttt gca ctg ggg aca gat ggg aaa aaa ttg agg atc aaa agc aag 4320
Phe Ala Leu Gly Thr Asp Gly Lys Lys Leu Arg Ile Lys Ser Lys
                1430                1435                1440

gag aaa tgc aat gat ggg aaa tgg cac acg gtg gtg ttt ggc cat 4365
Glu Lys Cys Asn Asp Gly Lys Trp His Thr Val Val Phe Gly His
                1445                1450                1455

gat ggg gaa aag ggg cgc ttg gtt gtg gat gga ctg agg gcc cgg 4410
Asp Gly Glu Lys Gly Arg Leu Val Val Asp Gly Leu Arg Ala Arg
                1460                1465                1470

gag gga agt ttg cct gga aac tcc acc atc agc atc aga gcg cca 4455
Glu Gly Ser Leu Pro Gly Asn Ser Thr Ile Ser Ile Arg Ala Pro
                1475                1480                1485

gtt tac ctg gga tca cct cca tca ggg aaa cca aag agc ctc ccc 4500
Val Tyr Leu Gly Ser Pro Pro Ser Gly Lys Pro Lys Ser Leu Pro
                1490                1495                1500

aca aac agc ttt gtg gga tgc ctg aag aac ttt cag ctg gat tca 4545
Thr Asn Ser Phe Val Gly Cys Leu Lys Asn Phe Gln Leu Asp Ser
                1505                1510                1515

aaa ccc ttg tat acc cct tct tca agc ttc ggg gtg tct tcc tgc 4590
Lys Pro Leu Tyr Thr Pro Ser Ser Ser Phe Gly Val Ser Ser Cys
                1520                1525                1530

ttg ggt ggt cct ttg gag aaa ggc att tat ttc tct gaa gaa gga 4635
Leu Gly Gly Pro Leu Glu Lys Gly Ile Tyr Phe Ser Glu Glu Gly
                1535                1540                1545

ggt cat gtc gtc ttg gct cac tct gta ttg ttg ggg cca gaa ttt 4680
Gly His Val Val Leu Ala His Ser Val Leu Leu Gly Pro Glu Phe
                1550                1555                1560

aag ctt gtt ttc agc atc cgc cca aga agt ctc act ggg atc cta 4725
Lys Leu Val Phe Ser Ile Arg Pro Arg Ser Leu Thr Gly Ile Leu
                1565                1570                1575

ata cac atc gga agt cag ccc ggg aag cac tta tgt gtt tac ctg 4770
Ile His Ile Gly Ser Gln Pro Gly Lys His Leu Cys Val Tyr Leu
                1580                1585                1590

gag gca gga aag gtc acg gcc tct atg gac agt ggg gca ggt ggg 4815
Glu Ala Gly Lys Val Thr Ala Ser Met Asp Ser Gly Ala Gly Gly
                1595                1600                1605

acc tca acg tcg gtc aca cca aag cag tct ctg tgt gat gga cag 4860
Thr Ser Thr Ser Val Thr Pro Lys Gln Ser Leu Cys Asp Gly Gln
                1610                1615                1620

tgg cac tcg gtg gca gtc acc ata aaa caa cac atc ctg cac ctg 4905
Trp His Ser Val Ala Val Thr Ile Lys Gln His Ile Leu His Leu
                1625                1630                1635

gaa ctg gac aca gac agt agc tac aca gct gga cag atc ccc ttc 4950
Glu Leu Asp Thr Asp Ser Ser Tyr Thr Ala Gly Gln Ile Pro Phe
                1640                1645                1650

cca cct gcc agc act caa gag cca cta cac ctt gga ggt gct cca 4995
Pro Pro Ala Ser Thr Gln Glu Pro Leu His Leu Gly Gly Ala Pro
                1655                1660                1665

gcc aat ttg acg aca ctg agg atc cct gtg tgg aaa tca ttc ttt 5040
Ala Asn Leu Thr Thr Leu Arg Ile Pro Val Trp Lys Ser Phe Phe
                1670                1675                1680

ggc tgt ctg agg aat att cat gtc aat cac atc cct gtc cct gtc 5085
Gly Cys Leu Arg Asn Ile His Val Asn His Ile Pro Val Pro Val
                1685                1690                1695
act gaa gcc ttg gaa gtc cag ggg cct gtc agt ctg aat ggt tgt 5130
Thr Glu Ala Leu Glu Val Gln Gly Pro Val Ser Leu Asn Gly Cys
                1700                1705                1710

cct gac cag 5139
Pro Asp Gln
1713
taacccaagc ctatttcaca gcaaggaaat tcaccttcaa aagcactgat 5189
tacccaatgc acctccctcc ccagctcgag atcattcttc aattaggaca 5239
caaaccagac aggtttaata gcgaatctaa ttttgaattc tgaccatgga 5289
tacccatcac tttggcattc agtgctacat gtgtatttta tataaaaatc 5339
ccatttcttg aagataaaaa aattgttatt caaattgtta tgcacagaat 5389
gtttttggta atattaattt ccactaaaaa attaaatgtc tttt 5433

<210> 2
<211> 1713
<212> PRT
<213> Human

<400> 2
Met Gly Trp Leu Trp Ile Phe Gly Ala Ala Leu Gly Gln Cys Leu
1               5                   10                  15
Gly Tyr Ser Ser Gln Gln Gln Arg Val Pro Phe Leu Gln Pro Pro
                20                  25                  30
Gly Gln Ser Gln Leu Gln Ala Ser Tyr Val Glu Phe Arg Pro Ser
                35                  40                  45
Gln Gly Cys Ser Pro Gly Tyr Tyr Arg Asp His Lys Gly Leu Tyr
                50                  55                  60
Thr Gly Arg Cys Val Pro Cys Asn Cys Asn Gly His Ser Asn Gln
                65                  70                  75
Cys Gln Asp Gly Ser Gly Ile Cys Val Asn Cys Gln His Asn Thr
                80                  85                  90
Ala Gly Glu His Cys Glu Arg Cys Gln Glu Gly Tyr Tyr Gly Asn
                95                  100                 105
Ala Val His Gly Ser Cys Arg Ala Cys Pro Cys Pro His Thr Asn
                110                 115                 120
Ser Phe Ala Thr Gly Cys Val Val Asn Gly Gly Asp Val Arg Cys
                125                 130                 135
Ser Cys Lys Ala Gly Tyr Thr Gly Thr Gln Cys Glu Arg Cys Ala
                140                 145                 150
Pro Gly Tyr Phe Gly Asn Pro Gln Lys Phe Gly Gly Ser Cys Gln
                155                 160                 165
Pro Cys Ser Cys Asn Ser Asn Gly Gln Leu Gly Ser Cys His Pro
                170                 175                 180
Leu Thr Gly Asp Cys Ile Asn Gln Glu Pro Lys Asp Ser Ser Pro
                185                 190                 195
Ala Glu Glu Cys Asp Asp Cys Asp Ser Cys Val Met Thr Leu Leu
                200                 205                 210
Asn Asp Leu Ala Thr Met Gly Glu Gln Leu Arg Leu Val Lys Ser

                215                 220                 225
Gln Leu Gln Gly Leu Ser Ala Ser Ala Gly Leu Leu Glu Gln Met
                230                 235                 240
Arg His Met Glu Thr Gln Ala Lys Asp Leu Arg Asn Gln Leu Leu
                245                 250                 255
Asn Tyr Arg Ser Ala Ile Ser Asn His Gly Ser Lys Ile Glu Gly
                260                 265                 270
Leu Glu Arg Glu Leu Thr Asp Leu Asn Gln Glu Phe Glu Thr Leu
                275                 280                 285
Gln Glu Lys Ala Gln Val Asn Ser Arg Lys Ala Gln Thr Leu Asn
                290                 295                 300
Asn Asn Val Asn Arg Ala Thr Gln Ser Ala Lys Glu Leu Asp Val
                305                 310                 315
Lys Ile Lys Asn Val Ile Arg Asn Val His Ile Leu Leu Lys Gln
                320                 325                 330
Ile Ser Gly Thr Asp Gly Glu Gly Asn Asn Val Pro Ser Gly Asp
                335                 340                 345
Phe Ser Arg Glu Trp Ala Glu Ala Gln Arg Met Met Arg Glu Leu
                350                 355                 360
Arg Asn Arg Asn Phe Gly Lys His Leu Arg Glu Ala Glu Ala Asp
                365                 370                 375
Lys Arg Glu Ser Gln Leu Leu Leu Asn Arg Ile Arg Thr Trp Gln
                380                 385                 390
Lys Thr His Gln Gly Glu Asn Asn Gly Leu Ala Asn Ser Ile Arg
                395                 400                 405
Asp Ser Leu Asn Glu Tyr Glu Ala Lys Leu Ser Asp Leu Arg Ala
                410                 415                 420
Arg Leu Gln Glu Ala Ala Ala Gln Ala Lys Gln Ala Asn Gly Leu
                425                 430                 435
Asn Gln Glu Asn Glu Arg Ala Leu Gly Ala Ile Gln Arg Gln Val
                440                 445                 450
Lys Glu Ile Asn Ser Leu Gln Ser Asp Phe Thr Lys Tyr Leu Thr
                455                 460                 465
Thr Ala Asp Ser Ser Leu Leu Gln Thr Asn Ile Ala Leu Gln Leu
                470                 475                 480
Met Glu Lys Ser Gln Lys Glu Tyr Glu Lys Leu Ala Ala Ser Leu
                485                 490                 495
Asn Glu Ala Arg Gln Glu Leu Ser Asp Lys Val Arg Glu Leu Ser
                500                 505                 510
Arg Ser Ala Gly Lys Thr Ser Leu Val Glu Glu Ala Glu Lys His
                515                 520                 525
Ala Arg Ser Leu Gln Glu Leu Ala Lys Gln Leu Glu Glu Ile Lys
                530                 535                 540
Arg Asn Ala Ser Gly Asp Glu Leu Val Arg Cys Ala Val Asp Ala
                545                 550                 555
Ala Thr Ala Tyr Glu Asn Ile Leu Asn Ala Ile Lys Ala Ala Glu
                560                 565                 570
Asp Ala Ala Asn Arg Ala Ala Ser Ala Ser Glu Ser Ala Leu Gln
                575                 580                 585
Thr Val Ile Lys Glu Asp Leu Pro Arg Lys Ala Lys Thr Leu Ser
                590                 595                 600
Ser Asn Ser Asp Lys Leu Leu Asn Glu Ala Lys Met Thr Gln Lys
                605                 610                 615
Lys Leu Lys Gln Glu Val Ser Pro Ala Leu Asn Asn Leu Gln Gln
                620                 625                 630
Thr Leu Asn Ile Val Thr Val Gln Lys Glu Val Ile Asp Thr Asn
                635                 640                 645
Leu Thr Thr Leu Arg Asp Gly Leu His Gly Ile Gln Arg Gly Asp
                650                 655                 660
Ile Asp Ala Met Ile Ser Ser Ala Lys Ser Met Val Arg Lys Ala
                665                 670                 675
Asn Asp Ile Thr Asp Glu Val Leu Asp Gly Leu Asn Pro Ile Gln
                680                 685                 690
Thr Asp Val Glu Arg Ile Lys Asp Thr Tyr Gly Arg Thr Gln Asn
                695                 700                 705
Glu Asp Phe Lys Lys Ala Leu Thr Asp Ala Asp Asn Ser Val Asn
                710                 715                 720
Lys Leu Thr Asn Lys Leu Pro Asp Leu Trp Arg Lys Ile Glu Ser
                725                 730                 735
Ile Asn Gln Gln Leu Leu Pro Leu Gly Asn Ile Ser Asp Asn Met
                740                 745                 750
Asp Arg Ile Arg Glu Leu Ile Gln Gln Ala Arg Asp Ala Ala Ser
                755                 760                 765
Lys Val Ala Val Pro Met Arg Phe Asn Gly Lys Ser Gly Val Glu
                770                 775                 780
Val Arg Leu Pro Asn Asp Leu Glu Asp Leu Lys Gly Tyr Thr Ser

                785                 790                 795
Leu Ser Leu Phe Leu Gln Arg Pro Asn Ser Arg Glu Asn Gly Gly
                800                 805                 810
Thr Glu Asn Met Phe Val Met Tyr Leu Gly Asn Lys Asp Ala Ser
                815                 820                 825
Arg Asp Tyr Ile Gly Met Ala Val Val Asp Gly Gln Leu Thr Cys
                830                 835                 840
Val Tyr Asn Leu Gly Asp Arg Glu Ala Glu Leu Gln Val Asp Gln
                845                 850                 855
Ile Leu Thr Lys Ser Glu Thr Lys Glu Ala Val Met Asp Arg Val
                860                 865                 870
Lys Phe Gln Arg Ile Tyr Gln Phe Ala Arg Leu Asn Tyr Thr Lys
                875                 880                 885
Gly Ala Thr Ser Ser Lys Pro Glu Thr Pro Gly Val Tyr Asp Met
                890                 895                 900
Asp Gly Arg Asn Ser Asn Thr Leu Leu Asn Leu Asp Pro Glu Asn
                905                 910                 915
Val Val Phe Tyr Val Gly Gly Tyr Pro Pro Asp Phe Lys Leu Pro
                920                 925                 930
Ser Arg Leu Ser Phe Pro Pro Tyr Lys Gly Cys Ile Glu Leu Asp
                935                 940                 945
Asp Leu Asn Glu Asn Val Leu Ser Leu Tyr Asn Phe Lys Lys Thr
                950                 955                 960
Phe Asn Leu Asn Thr Thr Glu Val Glu Pro Cys Arg Arg Arg Lys
                965                 970                 975
Glu Glu Ser Asp Lys Asn Tyr Phe Glu Gly Thr Gly Tyr Ala Arg
                980                 985                 990
Val Pro Thr Gln Pro His Ala Pro Ile Pro Thr Phe Gly Gln Thr
                995                 1000                1005
Ile Gln Thr Thr Val Asp Arg Gly Leu Leu Phe Phe Ala Glu Asn
                1010                1015                1020
Gly Asp Arg Phe Ile Ser Leu Asn Ile Glu Asp Gly Lys Leu Met
                1025                1030                1035
Val Arg Tyr Lys Leu Asn Ser Glu Leu Pro Lys Glu Arg Gly Val
                1040                1045                1050
Gly Asp Ala Ile Asn Asn Gly Arg Asp His Ser Ile Gln Ile Lys
                1055                1060                1065
Ile Gly Lys Leu Gln Lys Arg Met Trp Ile Asn Val Asp Val Gln
                1070                1075                1080
Asn Thr Ile Ile Asp Gly Glu Val Phe Asp Phe Ser Thr Tyr Tyr
                1085                1090                1095
Leu Gly Gly Ile Pro Ile Ala Ile Arg Glu Arg Phe Asn Ile Ser
                1100                1105                1110
Thr Pro Ala Phe Arg Gly Cys Met Lys Asn Leu Lys Lys Thr Ser
                1115                1120                1125
Gly Val Val Arg Leu Asn Asp Thr Val Gly Val Thr Lys Lys Cys
                1130                1135                1140
Ser Glu Asp Trp Lys Leu Val Arg Ser Ala Ser Phe Ser Arg Gly
                1145                1150                1155
Gly Gln Leu Ser Phe Thr Asp Leu Gly Leu Pro Pro Thr Asp His
                1160                1165                1170
Leu Gln Ala Ser Phe Gly Phe Gln Thr Phe Gln Pro Ser Gly Ile
                1175                1180                1185
Leu Leu Asp His Gln Thr Trp Thr Arg Asn Leu Gln Val Thr Leu
                1190                1195                1200
Glu Asp Gly Tyr Ile Glu Leu Ser Thr Ser Asp Ser Gly Gly Pro
                1205                1210                1215
Ile Phe Lys Ser Pro Gln Thr Tyr Met Asp Gly Leu Leu His Tyr
                1220                1225                1230
Val Ser Val Ile Ser Asp Asn Ser Gly Leu Arg Leu Leu Ile Asp
                1235                1240                1245
Asp Gln Leu Leu Arg Asn Ser Lys Arg Leu Lys His Ile Ser Ser
                1250                1255                1260
Ser Arg Gln Ser Leu Arg Leu Gly Gly Ser Asn Phe Glu Gly Cys
                1265                1270                1275
Ile Ser Asn Val Phe Val Gln Arg Leu Ser Leu Ser Pro Glu Val
                1280                1285                1290
Leu Asp Leu Thr Ser Asn Ser Leu Lys Arg Asp Val Ser Leu Gly
                1295                1300                1305
Gly Cys Ser Leu Asn Lys Pro Pro Phe Leu Met Leu Leu Lys Gly
                1310                1315                1320
Ser Thr Arg Phe Asn Lys Thr Lys Thr Phe Arg Ile Asn Gln Leu
                1325                1330                1335
Leu Gln Asp Thr Pro Val Ala Ser Pro Arg Ser Val Lys Val Trp
                1340                1345                1350
Gln Asp Ala Cys Ser Pro Leu Pro Lys Thr Gln Ala Asn His Gly

1355                1360                1365
Ala Leu Gln Phe Gly Asp Ile Pro Thr Ser His Leu Leu Phe Lys
            1370                1375                1380
Leu Pro Gln Glu Leu Leu Lys Pro Arg Ser Gln Phe Ala Val Asp
            1385                1390                1395
Met Gln Thr Thr Ser Ser Arg Gly Leu Val Phe His Thr Gly Thr
            1400                1405                1410
Lys Asn Ser Phe Met Ala Leu Tyr Leu Ser Lys Gly Arg Leu Val
            1415                1420                1425
Phe Ala Leu Gly Thr Asp Gly Lys Lys Leu Arg Ile Lys Ser Lys
            1430                1435                1440
Glu Lys Cys Asn Asp Gly Lys Trp His Thr Val Val Phe Gly His
            1445                1450                1455
Asp Gly Glu Lys Gly Arg Leu Val Val Asp Gly Leu Arg Ala Arg
            1460                1465                1470
Glu Gly Ser Leu Pro Gly Asn Ser Thr Ile Ser Ile Arg Ala Pro
            1475                1480                1485
Val Tyr Leu Gly Ser Pro Pro Ser Gly Lys Pro Lys Ser Leu Pro
            1490                1495                1500
Thr Asn Ser Phe Val Gly Cys Leu Lys Asn Phe Gln Leu Asp Ser
            1505                1510                1515
Lys Pro Leu Tyr Thr Pro Ser Ser Ser Phe Gly Val Ser Ser Cys
            1520                1525                1530
Leu Gly Gly Pro Leu Glu Lys Gly Ile Tyr Phe Ser Glu Glu Gly
            1535                1540                1545
Gly His Val Val Leu Ala His Ser Val Leu Leu Gly Pro Glu Phe
            1550                1555                1560
Lys Leu Val Phe Ser Ile Arg Pro Arg Ser Leu Thr Gly Ile Leu
            1565                1570                1575
Ile His Ile Gly Ser Gln Pro Gly Lys His Leu Cys Val Tyr Leu
            1580                1585                1590
Glu Ala Gly Lys Val Thr Ala Ser Met Asp Ser Gly Ala Gly Gly
            1595                1600                1605
Thr Ser Thr Ser Val Thr Pro Lys Gln Ser Leu Cys Asp Gly Gln
            1610                1615                1620
Trp His Ser Val Ala Val Thr Ile Lys Gln His Ile Leu His Leu
            1625                1630                1635
Glu Leu Asp Thr Asp Ser Ser Tyr Thr Ala Gly Gln Ile Pro Phe
            1640                1645                1650
Pro Pro Ala Ser Thr Gln Glu Pro Leu His Leu Gly Gly Ala Pro
            1655                1660                1665
Ala Asn Leu Thr Thr Leu Arg Ile Pro Val Trp Lys Ser Phe Phe
            1670                1675                1680
Gly Cys Leu Arg Asn Ile His Val Asn His Ile Pro Val Pro Val
            1685                1690                1695
Thr Glu Ala Leu Glu Val Gln Gly Pro Val Ser Leu Asn Gly Cys
            1700                1705                1710
Pro Asp Gln
        1713

<210> 3
<211> 3930
<212> DNA
<213> Human

<220>
<221> CDS
<222> (121)...(3630)

<300>
<301> Gerecke,D.R., Wagman,D.W., Champliaud,M.F. and Burgeson,R.E.
<302> The complete primary structure for a novel laminin chain, the
      laminin B1k chain
<303> J. Biol. Chem.
<304> 269
<305> 15
<306> 11073-11080
<307> 1994

<400> 3
gggcgggagg aggactgtat ctctggatgc ctggggcctg gtttcagggc ctgatttatt 60
cctcttcctg ggagctcact caggaaaggt cctttctggg gatcacccca ttggctgaag 120
atgagaccat tcttcctctt gtgttttgcc ctgcctggcc tcctgcatgc ccaacaagcc 180
tgctcccgtg gggcctgcta tccacctgtt ggggacctgc ttgttgggag gacccggttt 240
ctccgagctt catctacctg tggactgacc aagcctgaga cctactgcac ccagtatggc 300
gagtggcaga tgaaatgctg caagtgtgac tccaggcagc ctcacaacta ctacagtcac 360

cgagtagaga atgtggcttc atcctccggc cccatgcgct ggtggcagtc ccagaatgat 420
gtgaaccctg tctctctgca gctggacctg gacaggagat tccagcttca agaagtcatg 480
atggagttcc gagggcccat gcctgccggc atgctgattg agcgctcctc agacttcggt 540
aagacctggc gagtgtacca gtacctggct gccgactgca cctccacctt ccctcgggtc 600
cgccagggtc ggcctcagag ctggcaggat gttcggtgcc agtccctgcc tcagaggcct 660
aatgcacgcc taaatggggg gaaggtccaa cttaacctta tggatttagt gtctgggatt 720
ccagcaactc aaagtcaaaa aattcaagag gtgggggaga tcacaaactt gagagtcaat 780
ttcaccaggc tggcccctgt gccccaaagg ggctaccacc ctcccagcgc ctactatgct 840
gtgtcccagc tccgtctgca ggggagctgc ttctgtcacg gccatgctga tcgctgcgca 900
cccaagcctg gggcctctgc aggctccacc gctgtgcagg tccacgatgt ctgcgtctgc 960
cagcacaaca ctgccggccc aaattgtgag cgctgtgcac ccttctacaa caaccggccc 1020
tggagaccgg cggagggcca ggacgcccat gaatgccaaa ggtgcgactg caatgggcac 1080
tcagagacat gtcactttga ccccgctgtg tttgccgcca gccagggggc atatggaggt 1140
gtgtgtgaca attgccggga ccacaccgaa ggcaagaact gtgagcggtg tcagctgcac 1200
tatttccgga accggcgccc gggagcttcc attcaggaga cctgcatctc ctgcgagtgt 1260
gatccggatg gggcagtcgc aggggctccc tgtgacccag tgaccgggca gtgtgtgtgc 1320
aaggagcatg tgcagggaga gcgctgtgac ctatgcaagc cgggcttcac tggactcacc 1380
tacgccaacc cgcgacggtg ccaccgctgt gactgcaaca tcctggggtc ccgggagatg 1440
ccgtgtgacg aggagagtgg gcgctgcctt tgtctgccca acgtggtggg tcccaaatgt 1500
gaccagtgtg ctccctacca ctggaagctg gccagtggcc agggctgtga accgtgtgcc 1560
tgcgacccgc acaactccct cagcccacag tgcaaccagt tcacagggca gtgcccctgt 1620
cgggaaggct ttggtggcct gatgtgcagc gctgcagcca tccgccagtg tccagaccgg 1680
acctatggag acgtggccac aggatgccga gcctgtgact gtgatttccg gggaacagag 1740
ggcccgggct gcgacaaggc atcaggccgc tgcctctgcc gccctggctt gaccggcccc 1800
cgctgtgacc agtgccagcg aggctactgc aatcgctacc cggtgtgcgt ggcctgccac 1860
ccttgcttcc agacctatga tgcggacctc cgggagcagg ccctgcgctt tggtagactc 1920
ccgaatgcca ccgccagcct gtggtcaggg cctgggctgg aggaccgtgg cctggcctcc 1980
cggatcctag atgcaaagag taagattgag cagatccgag cagttctcag cagccccgca 2040
gtcacagagc aggaggtggc tcaggtggcc agtgccatcc tctccctcag gcgaactctc 2100
cagggcctgc agctggatct gcccctggag gaggagacgt tgtcccttcc gagagacctg 2160
gagagtcttg acagaagctt caatggtctc cttactatgt atcagaggaa gagggagcag 2220
tttgaaaaaa taagcagtgc tgatccttca ggagccttcc ggatgctgag cacagcctac 2280
gagcagtcag cccaggctgc tcagcaggtc tccgacagct cgcgcctttt ggaccagctc 2340
agggacagcc ggagagaggc agagaggctg gtgcggcagg cgggaggagg aggaggcacc 2400
ggcagcccca agcttgtggc cctgaggttg gagatgtctt cgttgcctga cctgacaccc 2460
accttcaaca agctctgtgg caactccagg cagatggctt gcaccccaat atcatgccct 2520
ggtgagctat gtccccaaga caatggcaca gcctgtgcgt cccgctgcag gggtgtcctt 2580
cccagggccg gtggggcctt cttgatggcg gggcaggtgg ctgagcagct gcggggcttc 2640
aatgcccagc tccagcggac caggcagatg attagggcag ccgaggaatc tgcctcacag 2700
attcaatcca gtgcccagcg cttggagacc caggtgagcg ccagccgctc ccagatggag 2760
gaagatgtca gacgcacacg gctcctaatc cagcaggtcc gggacttcct aacagacccc 2820
gacactgatg cagccactat ccaggaggtc agcgaggccg tgctggccct gtggctgccc 2880
acagactcag ctactgttct gcagaagatg aatgagatcc aggccattgc agccaggctc 2940
cccaacgtgg acttggtgct gtcccagacc aagcaggaca ttgcgcgtgc ccgccggttg 3000
caggctgagg ctgaggaagc caggagccga gcccatgcag tggagggcca ggtggaggat 3060
gtggttggga acctgcggca ggggacagtg gcactgcagg aagctcagga caccatgcaa 3120
ggcaccagcc ggtcccttcg gcttatccag gacagggttg ctgaggttca gcaggtactg 3180
cggccagcag aaaagctggt gacaagcatg accaagcagc tgggtgactt ctggacacgg 3240
atggaggagc tccgccacca agcccggcag cagggggcag aggcagtcca ggcccagcag 3300
cttgcggaag gtgccagcga gcaggcattg agtgcccaag agggatttga gagaataaaa 3360
caaaagtatg ctgagttgaa ggaccggttg ggtcagagtt ccatgctggg tgagcagggt 3420
gcccggatcc agagtgtgaa gacagaggca gaggagctgt ttggggagac catggagatg 3480
atggacagga tgaaagacat ggagttggag ctgctgcggg gcagccaggc catcatgctg 3540
cgctcagcgg acctgacagg actggagaag cgtgtggagc agatccgtga ccacatcaat 3600
gggcgcgtgc tctactatgc cacctgcaag tgatgctaca cgttccagcc cgttgcccca 3660
ctcatctgcg cgctttgctt ttggttgggg ggcagattgg gttggaatgc tttccatctc 3720
caggagactt tcatgtagcc caaagtacag cctggaccac ccctggtgtg tgtagctagt 3780
aagattaccc tgagctgcag ctgagcctga gccaatggga cagttacact tgacagacaa 3840
agatggtgga gattggcatg ccattgaaac taagagctct caagtcaagg aagctgggct 3900
gggcagtatc cccgcctttt agttctccac 3930

<210> 4
<211> 1170
<212> PRT
<213> Human

<400> 4

<210> 5
<211> 5200
<212> DNA
<213> Human

<220>
<221> CDS
<222> (118)...(3696)

<300>
<301> Kallunki,P., Sainio,K., Eddy,R., Byers,M., Kallunki,T., Sariola,H.,
      Beck,K., Hirvonen,H., Shows,T.B. and Tryggvason,K.
<302> A truncated laminin chain homologous to the B2 chain: structure,
      spatial expression, and chromosomal assignment
<303> J. Cell Biol.
<304> 119
<306> 679-693
<307> 1992

<400> 5
   gaccacctga tcgaaggaaa aggaaggcac agcggagcgc agagtgagaa ccaccaaccg 60
   aggcgccggg cagcgacccc tgcagcggag acagagactg agcggcccgg caccgccatg 120
   cctgcgctct ggctgggctg ctgcctctgc ttctcgctcc tcctgcccgc agcccgggcc 180
   acctccagga gggaagtctg tgattgcaat gggaagtcca ggcagtgtat ctttgatcgg 240
   gaacttcaca gacaaactgg taatggattc cgctgcctca actgcaatga caacactgat 300
   ggcattcact gcgagaagtg caagaatggc ttttaccggc acagagaaag ggaccgctgt 360
   ttgccctgca attgtaactc caaaggttct cttagtgctc gatgtgacaa ctctggacgg 420
   tgcagctgta aaccaggtgt gacaggagcc agatgcgacc gatgtctgcc aggcttccac 480
   atgctcacgg atgcggggtg cacccaagac cagagactgc tagactccaa gtgtgactgt 540
   gacccagctg gcatcgcagg gccctgtgac gcgggccgct gtgtctgcaa gccagctgtt 600
   actggagaac gctgtgatag gtgtcgatca ggttactata atctggatgg ggggaaccct 660
   gagggctgta cccagtgttt ctgctatggg cattcagcca gctgccgcag ctctgcagaa 720
   tacagtgtcc ataagatcac ctctaccttt catcaagatg ttgatggctg gaaggctgtc 780
   caacgaaatg ggtctcctgc aaagctccaa tggtcacagc gccatcaaga tgtgtttagc 840
   tcagcccaac gactagatcc tgtctatttt gtggctcctg ccaaatttct tgggaatcaa 900
   caggtgagct atgggcaaag cctgtccttt gactaccgtg tggacagagg aggcagacac 960
   ccatctgccc atgatgtgat cctggaaggt gctggtctac ggatcacagc tcccttgatg 1020
   ccacttggca agacactgcc ttgtgggctc accaagactt acacattcag gttaaatgag 1080
   catccaagca ataattggag cccccagctg agttactttg agtatcgaag gttactgcgg 1140
   aatctcacag ccctccgcat ccgagctaca tatggagaat acagtactgg gtacattgac 1200
   aatgtgaccc tgatttcagc ccgccctgtc tctggagccc cagcaccctg ggttgaacag 1260
   tgtatatgtc ctgttgggta caaggggcaa ttctgccagg attgtgcttc tggctacaag 1320
   agagattcag cgagactggg gccttttggc acctgtattc cttgtaactg tcaaggggga 1380
   ggggcctgtg atccagacac aggagattgt tattcagggg atgagaatcc tgacattgag 1440
   tgtgctgact gcccaattgg tttctacaac gatccgcacg acccccgcag ctgcaagcca 1500
   tgtccctgtc ataacgggtt cagctgctca gtgattccgg agacggagga ggtggtgtgc 1560
   aataactgcc ctcccggggt caccggtgcc cgctgtgagc tctgtgctga tggctacttt 1620
   ggggacccct ttggtgaaca tggcccagtg aggccttgtc agccctgtca atgcaacagc 1680
   aatgtggacc ccagtgcctc tgggaattgt gaccggctga caggcaggtg tttgaagtgt 1740
   atccacaaca cagccggcat ctactgcgac cagtgcaaag caggctactt cggggaccca 1800
   ttggctccca acccagcaga caagtgtcga gcttgcaact gtaaccccat gggctcagag 1860
   cctgtaggat gtcgaagtga tggcacctgt gtttgcaagc caggatttgg tggccccaac 1920
   tgtgagcatg gagcattcag ctgtccagct tgctataatc aagtgaagat tcagatggat 1980
   cagtttatgc agcagcttca gagaatggag gccctgattt caaaggctca gggtggtgat 2040
   ggagtagtac ctgatacaga gctggaaggc aggatgcagc aggctgagca ggcccttcag 2100
   gacattctga gagatgccca gatttcagaa ggtgctagca gatcccttgg tctccagttg 2160
   gccaaggtga ggagccaaga gaacagctac cagagccgcc tggatgacct caagatgact 2220
   gtggaaagag ttcgggctct gggaagtcag taccagaacc gagttcggga tactcacagg 2280
   ctcatcactc agatgcagct gagcctggca gaaagtgaag cttccttggg aaacactaac 2340
   attcctgcct cagaccacta cgtgggccca aatggcttta aaagtctggc tcaggaggcc 2400
   acaagattag cagaaagcca cgttgagtca gccagtaaca tggagcaact gacaaggaaa 2460
   actgaggact attccaaaca agccctctca ctggtgcgca aggccctgca tgaaggagtc 2520
   ggaagcggaa gcggtagccc ggacggtgct gtggtgcaag ggcttgtgga aaaattggag 2580
   aaaaccaagt ccctggccca gcagttgaca agggaggcca ctcaagcgga aattgaagca 2640
   gataggtctt atcagcacag tctccgcctc ctggattcag tgtctccgct tcagggagtc 2700
   agtgatcagt cctttcaggt ggaagaagca aagaggatca aacaaaaagc ggattcactc 2760
   tcaagcctgg taaccaggca tatggatgag ttcaagcgta cacaaaagaa tctgggaaac 2820
   tggaaagaag aagcacagca gctcttacag aatggaaaaa gtgggagaga gaaatcagat 2880
   cagctgcttt cccgtgccaa tcttgctaaa agcagagcac aagaagcact gagtatgggc 2940
   aatgccactt tttatgaagt tgagagcatc cttaaaaacc tcagagagtt tgacctgcag 3000
   gtggacaaca gaaaagcaga agctgaagaa gccatgaaga gactctccta catcagccag 3060
   aaggtttcag atgccagtga caagacccag caagcagaaa gagccctggg gagcgctgct 3120
   gctgatgcac agagggcaaa gaatggggcc ggggaggccc tggaaatctc cagtgagatt 3180
   gaacaggaga ttgggagtct gaacttggaa gccaatgtga cagcagatgg agccttggcc 3240
   atggaaaagg gactggcctc tctgaagagt gagatgaggg aagtggaagg agagctggaa 3300
   aggaaggagc tggagtttga cacgaatatg gatgcagtac agatggtgat tacagaagcc 3360
   cagaaggttg ataccagagc caagaacgct ggggttacaa tccaagacac actcaacaca 3420
   ttagacggcc tcctgcatct gatggaccag cctctcagtg tagatgaaga ggggctggtc 3480
   ttactggagc agaagctttc ccgagccaag acccagatca acagccaact gcggcccatg 3540
   atgtcagagc tggaagagag ggcacgtcag cagaggggcc acctccattt gctggagaca 3600
   agcatagatg ggattctggc tgatgtgaag aacttggaga acattaggga caacctgccc 3660
   ccaggctgct acaataccca ggctcttgag caacagtgaa gctgccataa atatttctca 3720
   actgaggttc ttgggataca gatctcaggg ctcgggagcc atgtcatgtg agtgggtggg 3780
   atggggacat ttgaacatgt ttaatgggta tgctcaggtc aactgacctg accccattcc 3840
   tgatcccatg gccaggtggt tgtcttattg caccatactc cttgcttcct gatgctgggc 3900
   atgaggcaga taggcactgg tgtgagaatg atcaaggatc tggaccccaa agatagactg 3960
   gatggaaaga caaactgcac aggcagatgt ttgcctcata atagtcgtaa gtggagtcct 4020
   ggaatttgga caagtgctgt tgggatatag tcaacttatt ctttgagtaa tgtgactaaa 4080
   ggaaaaaact ttgactttgc ccaggcatga aattcttcct aatgtcagaa cagagtgcaa 4140
   cccagtcaca ctgtggccag taaaatacta ttgcctcata ttgtcctctg caagcttctt 4200
   gctgatcaga gttcctccta cttacaaccc agggtgtgaa catgttctcc attttcaagc 4260
   tggaagaagt gagcagtgtt ggagtgagga cctgtaaggc aggcccattc agagctatgg 4320
   tgcttgctgg tgcctgccac cttcaagttc tggacctggg catgacatcc tttcttttaa 4380
   tgatgccatg gcaacttaga gattgcattt ttattaaagc atttcctacc agcaaagcaa 4440
   atgttgggaa agtatttact ttttcggttt caaagtgata gaaaagtgtg gcttgggcat 4500
   tgaaagaggt aaaattctct agatttatta gtcctaattc aatcctactt ttcgaacacc 4560
   aaaaatgatg cgcatcaatg tattttatct tattttctca atctcctctc tctttcctcc 4620
   acccataata agagaatgtt cctactcaca cttcagctgg gtcacatcca tccctccatt 4680
   catccttcca tccatctttc catccattac ctccatccat ccttccaaca tatatttatt 4740
   gagtacctac tgtgtgccag gggctggtgg gacagtggtg acatagtctc tgccctcata 4800
   gagttgattg tctagtgagg aagacaagca tttttaaaaa ataaatttaa acttacaaac 4860
   tttgtttgtc acaagtggtg tttattgcaa taaccgcttg gtttgcaacc tctttgctca 4920
   acagaacata tgttgcaaga ccctcccatg ggcactgagt ttggcaagga tgacagagct 4980
   ctgggttgtg cacatttctt tgcattccag cgtcactctg tgccttctac aactgattgc 5040
   aacagactgt tgagttatga taacaccagt gggaattgct ggaggaacca gaggcacttc 5100
   caccttggct gggaagacta tggtgctgcc ttgcttctgt atttccttgg attttcctga 5160
   aagtgttttt aaataaagaa caattgttag atgccaaaaa 5200

<210> 6
<211> 1193
<212> PRT
<213> Human

<400> 6

<210> 7
<211> 20
<212> DNA
<213> Artificial Sequence

<220>
<223> primer for PCR amplification

<400> 7
aagcagaaga tgcggactgt    20

<210> 8
<211> 20
<212> DNA
<213> Artificial Sequence

<220>
<223> primer for PCR amplification

<400> 8
accactggtt tttctgccac    20

<210> 9
<211> 23
<212> DNA
<213> Artificial Sequence

<220>
<223> primer for PCR amplification

<400> 9
tctttccacc aggcccccgg ctc    23

<210> 10
<211> 23
<212> DNA
<213> Artificial Sequence

<220>
<223> primer for PCR amplification

<400> 10
tgcgggcgga catggggaga tcc    23

<210> 11
<211> 23
<212> DNA
<213> Artificial Sequence

<220>
<223> primer for PCR amplification

<400> 11
tagagctaga ctccgggcga tga    23

<210> 12
<211> 23
<212> DNA
<213> Artificial Sequence

<220>
<223> primer for PCR amplification

<400> 12
ttgccttaaa caagaccacg aaa    23

<210> 13
<211> 20
<212> DNA
<213> Artificial Sequence

<220>
<223> primer for PCR amplification

<400> 13
caccatggag aaggccgggg    20

<210> 14
<211> 20
<212> DNA
<213> Artificial Sequence

<220>
<223> primer for PCR amplification

<400> 14
gacggacaca ttgggggtag    20

## Description

### TECHNICAL FIELD

The present invention relates to a method for culturing cells in a system containing laminin-5.

### BACKGROUND ART

Laminin, which is localized primarily on the basement membranes of various tissues, is an extracellular matrix protein playing an important role maintaining tissue structure and in controlling cell functions (Matrix Biol., 18:19-28, 1999, Dev. Dyn., 218:213-234, 2000).

Laminin is structured as a heterotrimer molecule composed of α, β and γ chains linked to each other via disulfide linkages, which takes a characteristic cross-structure. Each chain is composed of multiple domains, and domains I and II form a triple helix. Before the filing of the present application, at least 15 isoforms of laminin molecules have been identified from different combinations of 5 types of α chains (α1 to α5), 3 types of β chains (β1 to β3) and 3 types of γ chains (γ1 to γ3), and it is suggested that there are actually several times that number of isoforms (Cancer Sci., 97:91-98, 2006; Dev. Dyn., 218:213-234, 2000; J. Neurosci., 20:6517-6528, 2000; Physiol Rev. 85:979-1000, 2005). These α, β and γ chains are encoded by different genes, respectively; and the individual laminin isoforms have specific sites of localization and specific functions, and mainly regulate cell adhesion, proliferation, motility, differentiation and so on through the cell membrane receptor integrin (Dev. Dyn. 218:213-234, 2000, Physiol. Rev. 85:979-1000, 2005).

For example, concerning localization *in vivo,* α2 chains center around the muscle or the nervous tissue, whereas α3 chains center around the skin tissue. The chains differ in their functions as well; specifically, a gene abnormality in the α2 chain causes muscular dystrophy, whereas the gene abnormality in the α3 chain causes a serious symptom known as junctional epidermolysis bullosa (Dev. Dyn. 218:213-234, 2000). In addition, the chains exhibit completely different functions from each other in *an in vitro* experiment. Laminin 2 and laminin 4, which comprise an α2 chain as their component chain, exhibit almost no adhesion activity against mesenchymal stem cells, whereas laminin-5, which comprise an α3 chain as its component chain, exhibits extremely strong adhesion activity to such cells (Stem Cell. 24:2346-2354, 2006). Differences in the component α, β, γ chains lead to differences in the functions and activities of the laminin isoforms.

Fifteen laminin molecular species and the corresponding subunit arrangement of each species are shown in Table 1.

**[Table 1]**

| Laminin molecular species and subunit structure | | |
|---|---|---|
| Name | Structure | Also called |
| Laminin-1 | α1β1γ1 | EHS laminin |
| Laminin-2 | α1β2γ1 | Merosin |
| Laminin-3 | α1β2γ1 | S-Laminin |
| Laminin-4 | α2β2γ1 | S-Merosin |
| Laminin-5 | α3β3γ2 | Ladsin/epiligrin/kalinin/nicein |
| Laminin-6 | α3β31γ1 | K-Laminin |
| Laminin-7 | α3β2γ1 | KS-Laminin |
| Laminin-8 | α4β1γ1 | |
| Laminin-9 | α4β2γ1 | |
| Laminin-10 | α5β1γ1 | |
| Laminin-11 | α5β2γ1 | |
| Laminin-12 | α2β1γ3 | |
| Laminin-13 | α3β2γ3 | |
| Laminin-14 | α4β2γ3 | |
| Laminin-15 | α5β2γ3 | |

Laminin molecules form a basement membrane through associating with each other or to other matrix molecules by the amino (N) terminal sections (short arms) of the three chains. Meanwhile, the carboxy (C) terminal of the α chain comprises five homologous spherical domains (G1 - G5 domains or LG1 - LG5), as primary sites for bonding with integrin and other receptors.

### Laminin-5

Laminin-5 (also known as kalinin, epiligrin, nicein, ladsin), which is a laminin isoform composed of an α3 chain, a β3 chain and a γ2 chain, was discovered by different research institutes through different routes. (J. Cell Biol.114:567-576, 1991; Cell 65:599-610, 1991; J. Invest Dermatol. 101:738-743, 1993; Proc. Natl. Acad. Sci. USA 90:11767-11771, 1993).

Laminin-5 is reported to have strong cell adhesion activity, cell scattering activity, cell proliferation activity and the like against various cells (Proc. Natl. Acad. Sci. USA. 90:11767-11771, 1993; J. Biochem. 116:862-869, 1994; J. Cell Biol. 125:205-214, 1994; Mol. Biol. Cell. 16:881-890, 2005, Stem Cell. 24:2346-2354, 2006). WO 2007/023875 discloses culture techniques using laminin-5 for mesenchymal stem cells.

However, there was no general efficient method for enhancing various activities of laminin-5 before the present invention.

### CITATION LIST

### PATENT DOCUMENTS

[Patent Document 1] International Publication WO 2007/023875
[Patent Document 2] International Publication WO 2009/123349

### [Non-Patent Documents]

[Non-Patent Document 1] Matrix Biol., 18:19-28, 1999
[Non-Patent Document 2] Dev. Dyn., 218:213-234, 2000
[Non-Patent Document 3] Cancer Sci., 97:91-98, 2006
[Non-Patent Document 4] J. Neurosci., 20:6517-6528, 2000
[Non-Patent Document 5] Physiol. Rev.85:979-1000, 2005
[Non-Patent Document 6] J. Cell Biol.114:567-576, 1991
[Non-Patent Document 7] Cell. 65:599-610, 1991
[Non-Patent Document 8] J. Invest Dermatol.101:738-743, 1993
[Non-Patent Document 9] Proc. Natl. Acad. Sci. USA. 90:11767-11771, 1993
[Non-Patent Document 10] J. Biochem. 116:862-869, 1994
[Non-Patent Document 11] J. Cell Biol. 125:205-214,1994
[Non-Patent Document 12] Mol. Biol. Cell.16:881-890, 2005
[Non-Patent Document 13] Stem Cell. 24:2346-2354, 2006
[Non-Patent Document 14] Dev. Biol. 163: p. 288-292, 1994
[Non-Patent Document 15] J. Cell Sci. 112:1-10, 1999
[Non-Patent Document 16] Exp. Cell Res. 310:256-269, 2005
[Non-Patent Document 17] J. Cell Sci. 119:3206-3218, 2006

### SUMMARY OF INVENTION

### TECHNICAL PROBLEM

The object of the present invention is to provide a technology for increasing the activity of laminin-5 in a method for culturing cells in a system containing laminin-5.

The present inventors found that various activities of laminin-5 increase by the combined use of specific polypeptides with laminin-5 in a method for culturing cells in a system containing laminin-5, and conceived the present invention.

### SOLUTION TO PROBLEM

The present invention comprises the following preferable embodiments.
[Embodiment 1] A method for culturing cells in a system containing laminin-5 characterized by a culture system comprising a polypeptide selected from a group consisting of: a protein in blood other than extracellular matrix proteins, which is, serum, serum albumin, prealbumin, immunoglobulin, α-globulin, β-globulin, α1-antitrypsin (α1-AT), heptoglobin (Hp), α2-macroglobulin (α2-M), α-fetoprotein (AFP), transferrin, retinol-binding protein (RBP) or adiponectin; gelatin; a protein belonging to a tumor necrosis factor (TNF) family; and peptone.
[Embodiment 2] The method according to Embodiment 1, wherein the protein belonging to the tumor necrosis factor (TNF) family is a receptor activator NF_{κ}B ligand (RANKL).
[Embodiment 3] The method according to Embodiment 1, wherein peptone is selected from a group consisting of a cotton seed derived peptone, a soy bean derived peptone, a wheat derived peptone and a pea derived peptone.
[Embodiment 4] The method according to any one of Embodiments 1 to 3, wherein a cell culture vessel is treated with laminin-5 after it is treated with polypeptide, or the vessel is treated with polypeptide and laminin at the same time.
[Embodiment 5] The method according to any one of Embodiments 1 to 4, wherein an activity of laminin-5 against the cells, selected from a group consisting of a cell adhesion activity, a cell scattering activity, a wound healing activity, a proliferation stimulating activity, an activity for maintaining undifferentiated-state and an activity for maintaining pluripotency is increased.
[Embodiment 6] The method according to any one of Embodiments 1 to 5, wherein the cells are selected from a group consisting of pluripotent stem cells, tissue stem cells, somatic cells, germ cells and sacroma cells.
[Embodiment 7] The method according to Embodiment 6, wherein the pluripotent stem cells are selected from embryonic stem cells, induced pluripotent stem cells, embryonic germ cells or germline stem cells;
   the tissue stem cells are selected from mesenchymal stem cells, hepatic stem cells, pancreatic stem cells, neural stem cells, skin stem cells or hematopoietic stem cells; or
   the somatic cells are selected from hepatic cells, pancreatic cells, muscle cells, osteocytes, osteoblasts, osteoclasts, cartilage cells, fat cells, skin cells, fibroblasts, pancreatic cells, kidney cells, pneumocytes or blood cells, which are lymphocites, red blood cells, white blood cells, monocytes, macrophage or megakaryocytes.
[Embodiment 8] The method according to any one of Embodiments 1 to 5, wherein the cells are derived from a species selected from a group consisting of a mouse, a rat or a human.
[Embodiment 9] The method according to any one of Embodiments 1 to 8, wherein the polypeptide is used at a concentration of 1 µg/ml to 200 µg/ml.
[Embodiment 10] The method according to any one of Embodiments 1 to 8, wherein the polypeptide is used at a concentration of 3.125 µg/ml to 12.5 µg/ml,
[Embodiment 11] The method according to any one of Embodiments 1 to 8, wherein the cell culture system comprises two or more types of polypeptides.
[Embodiment 12] A composition to be used in a method for culturing cells in a system containing laminin-5, wherein the composition comprises a polypeptide selected from a group consisting of: a protein in blood other than extracellular matrix proteins, which is, serum, serum albumin, prealbumin, immunoglobulin, α-globulin, β-globulin, α1-antitrypsin (α1-AT), heptoglobin (Hp), α2-macroglobulin (α2-M), α-fetoprotein (AFP), transferrin, retinol-binding protein (RBP) or adiponectin; gelatin; a protein belonging to a tumor necrosis factor (TNF) family; and peptone.
[Embodiment 13] The composition according to Embodiment 12, wchich further comprises laminin-5.
[Embodiment 14] A kit for use in a method for culturing cells in a system containing laminin-5, wherein the system contains a polypeptide selected from a group consisting of: a protein in blood other than extracellular matrix proteins, which is, serum, serum albumin, prealbumin, immunoglobulin, α-globulin, β-globulin, α1-antitrypsin (α1-AT), heptoglobin (Hp), α2-macroglobulin (α2-M), α-fetoprotein (AFP), transferrin, retinol-binding protein (RBP) or adiponectin; gelatin; a protein belonging to a tumor necrosis factor (TNF) family; and peptone.
[Embodiment 15] The kit according to Embodiment 14 which further comprises laminin-5.

### ADVANTAGEOUS EFFECT OF INVENTION

In the present invention, a combined use of laminin-5 and specific polypeptides increases the activity of laminin-5 against a cell, which is selected from a group consisting of a cell adhesion activity, a cell scattering activity, a wound healing activity, a proliferation stimulating activity, an activity for maintaining undifferentiated-state and an activity for pluripotency.

### BRIEF DESCRIPTION OF DRAWINGS

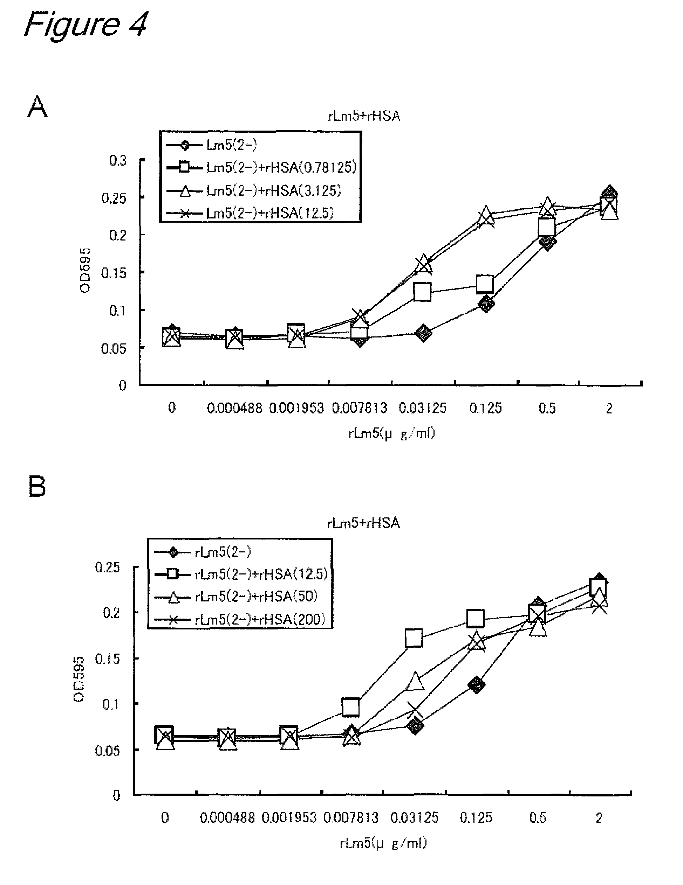

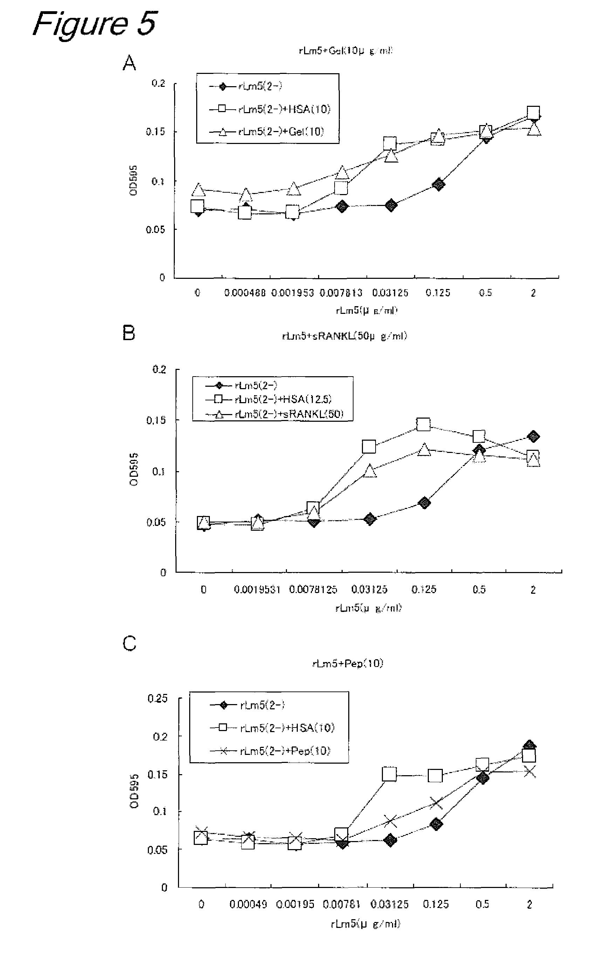

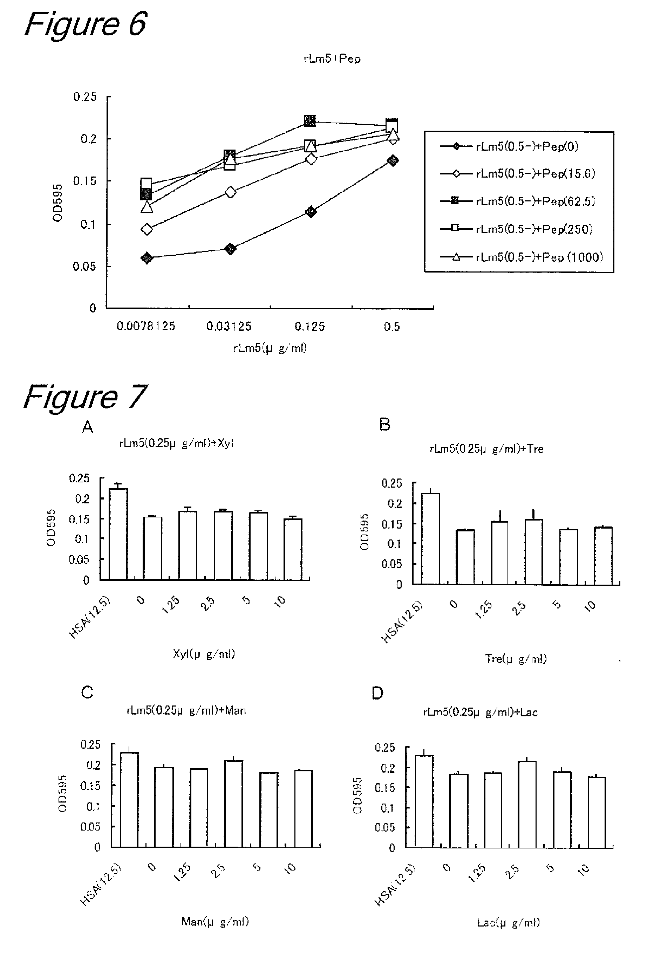

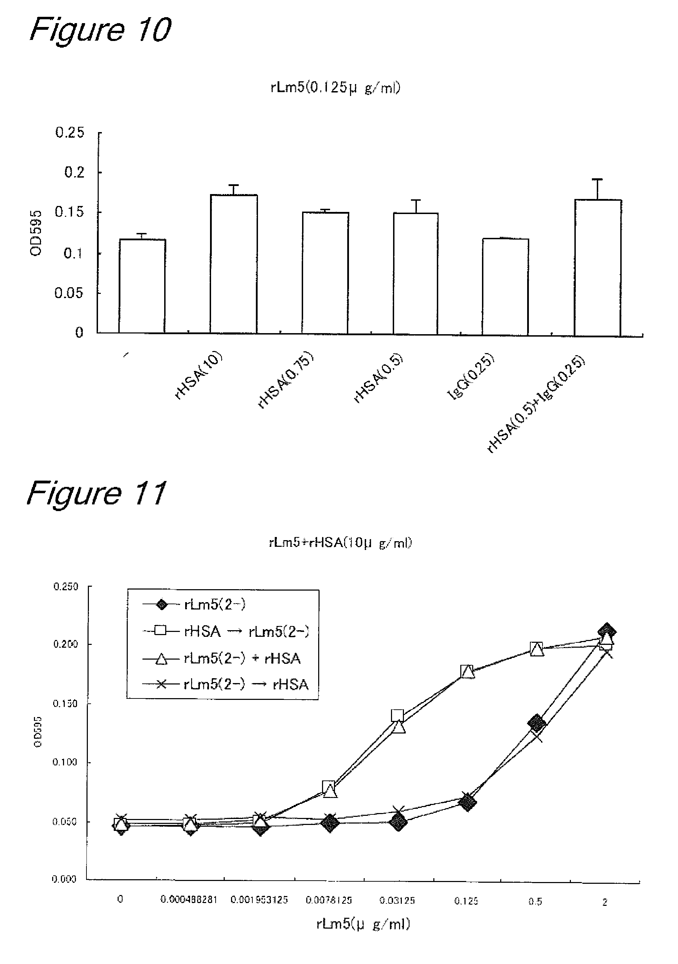

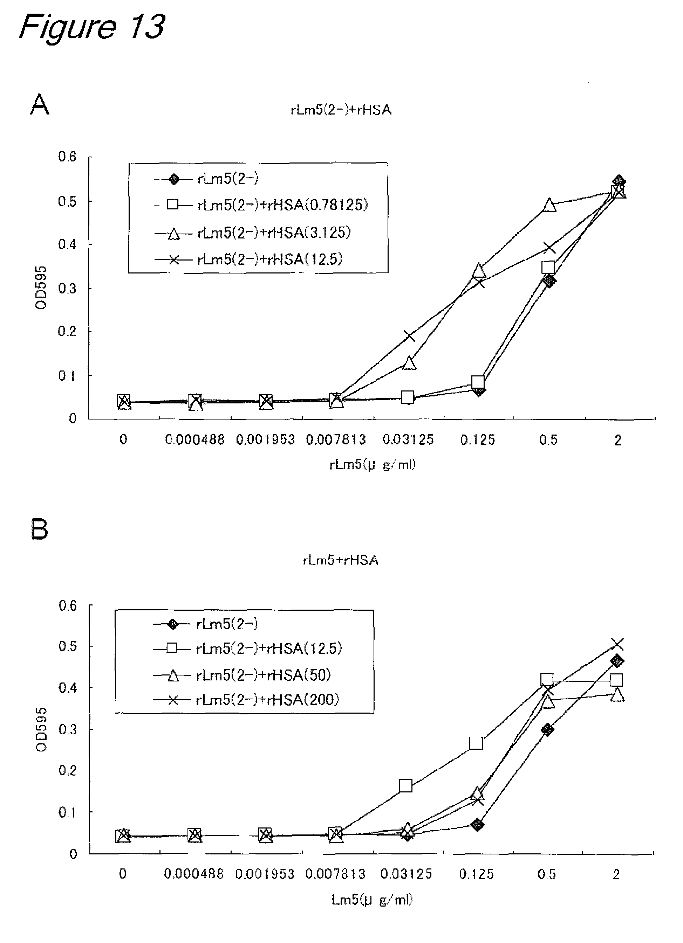

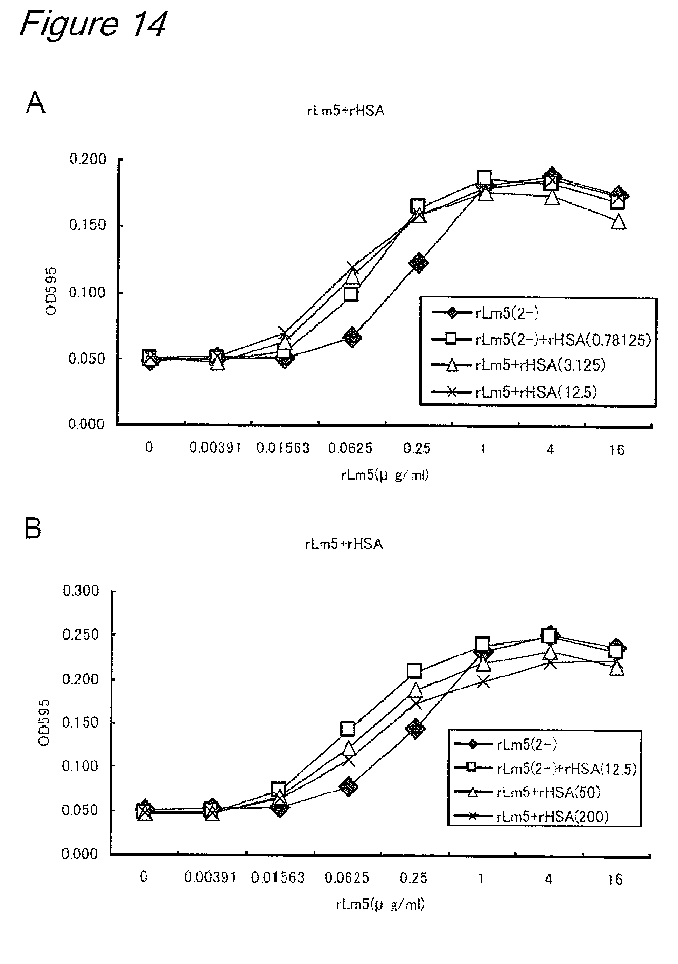

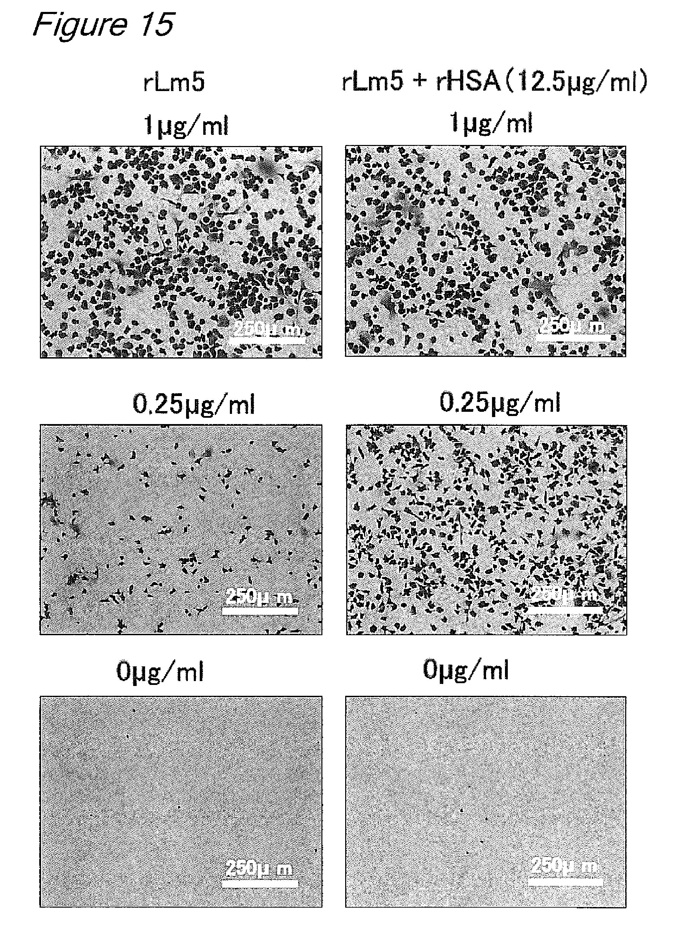

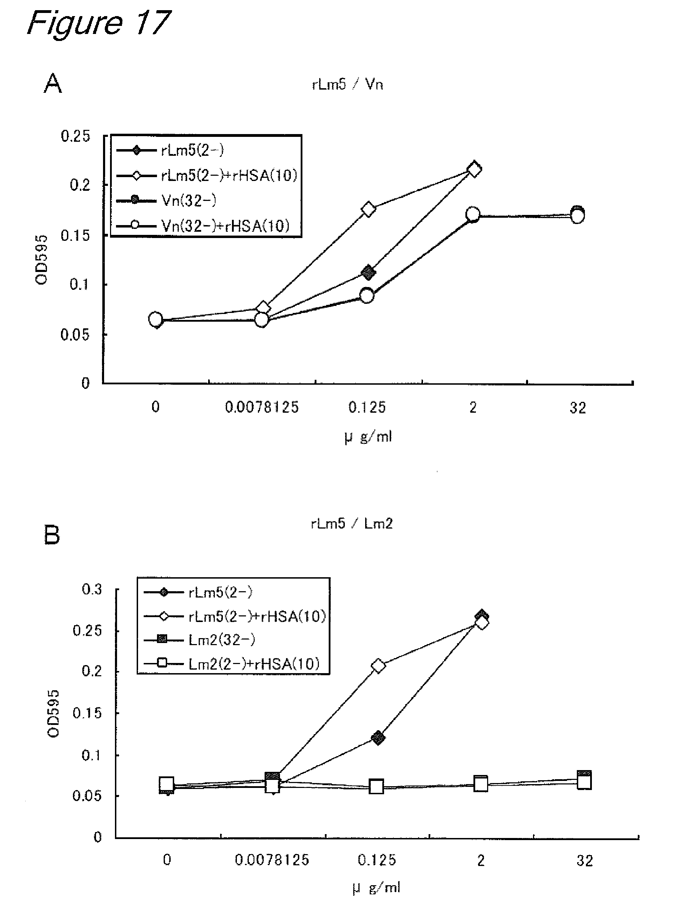

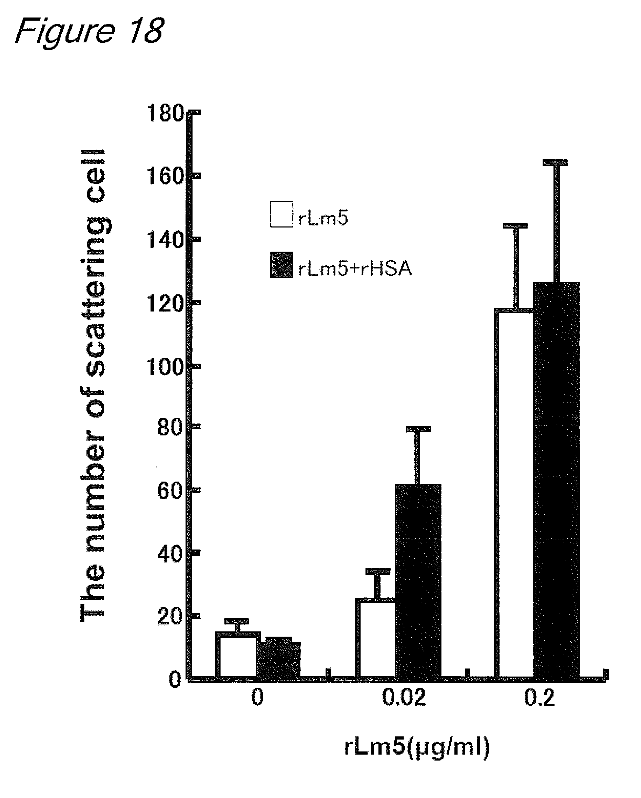

Figure 1 shows electrophoresis of purified recombinant human laminin-5 on an SDS polyacrylamide gel. It should be noted that the right lane in Figure 1 shows the results of electrophoresis of 1 µg of recombinant human laminin-5.
Figure 2 shows the effect of recombinant human laminin-5 (0.25 µg/ml) and various proteins in blood on the cell adhesion activity against BRL cells. Figures 2A-D show results for each of human serum albumin (HSA), bovine serum albumin (BSA), human serum (HS) and IgG, at the concentrations shown in the figures (0-800 µg/ml).
Figure 3 shows the result of an investigation for the optimal concentration of HSA to increase the cell adhesion activity of the recombinant human laminin-5 at concentrations provided on the x-axis (0-2 µg/ml) against BRL cells.
   Figure 3A
      Black diamond: just recombinant human laminin-5
      White square: recombinant human laminin-5 + HSA 0.78125 µg/ml
      White triangle: recombinant human laminin-5 + HSA 3.125 µg/ml
      Cross: recombinant human laminin-5 + HSA 12.5 µg/ml
   Figure 3B
      Black diamond: just recombinant human laminin-5
      White square: recombinant human laminin-5 + HSA 12.5 µg/ml
      White triangle: recombinant human laminin-5 + HSA 50 µg/ml
      Cross: recombinant human laminin-5 + HSA 200 µg/ml
Figure 4 shows the result of an investigation for the optimal concentration of recombinant HSA (rHSA) to increase the cell adhesion activity of the recombinant human laminin-5 at concentrations provided on the x-axis (0-2 µg/ml) against BRL cells.
   Figure 4A
      Black diamond: just recombinant human laminin-5
      White square: recombinant human laminin-5 + rHSA 0.78125 µg/ml
      White triangle: recombinant human laminin-5 + rHSA 3.125 µg/ml
      Cross: recombinant human laminin-5 + rHSA 12.5 µg/ml
   Figure 4B
      Black diamond: just recombinant human laminin-5
      White square: recombinant human laminin-5 + rHSA 12.5 µg/ml
      White triangle: recombinant human laminin-5 + rHSA 50 µg/ml
      Cross: recombinant human laminin-5 + rHSA 200 µg/ml
Figure 5 shows the result of an investigation on the elevating effects of gelatin (Gel), sRANKL and peptine (Pep) on the cell adhesion activity of the recombinant human laminin-5 at concentrations provided on the x-axis (0-2 µg/ml) against BRL cells.
   Figure 5A
      Black diamond: just recombinant human laminin-5
      White square: recombinant human laminin-5 + HSA 10 µg/ml
      White triangle: recombinant human laminin-5 + Gel 10 µg/ml
   Figure 5B
      Black diamond: just recombinant human laminin-5
      White square: recombinant human laminin-5 + HSA 1.2.5 µg/ml
      White triangle: recombinant human laminin-5 + sRANKL 50 µg/ml
   Figure 5C
      Black diamond: just recombinant human laminin-5
      White square: recombinant human laminin-5 + HSA 10 µg/ml
      White triangle: recombinant human laminin-5 + Pep 10 µg/ml
Figure 6 shows the result of an investigation on the elevating effects of Pep on the cell adhesion activity of the recombinant human laminin-5 at concentrations provided on the x-axis (0-2 µg/ml) against BRL cells.
   Black diamond: just recombinant human laminin-5
   White diamond: recombinant human laminin-5 + Pep 15.6 µg/ml
   Black square: recombinant human laminin-5 + Pep 62.5 µg/ml
   White square: recombinant human laminin-5 + Pep 250 µg/ml
   White triangle: recombinant human laminin-5 + Pep 1000 µg/ml
Figure 7 shows the result of an investigation on whether recombinant human laminin-5 (0.25 µg/ml) and various sugars have effects on the cell adhesion activity against BRL cells. Figures 7A-D respectively show the results, each for xylose (Xyl), trehalose (Tre), mannose (Man) and lactose (Lac) at the concentrations shown in the figures (0-10 µg/ml).

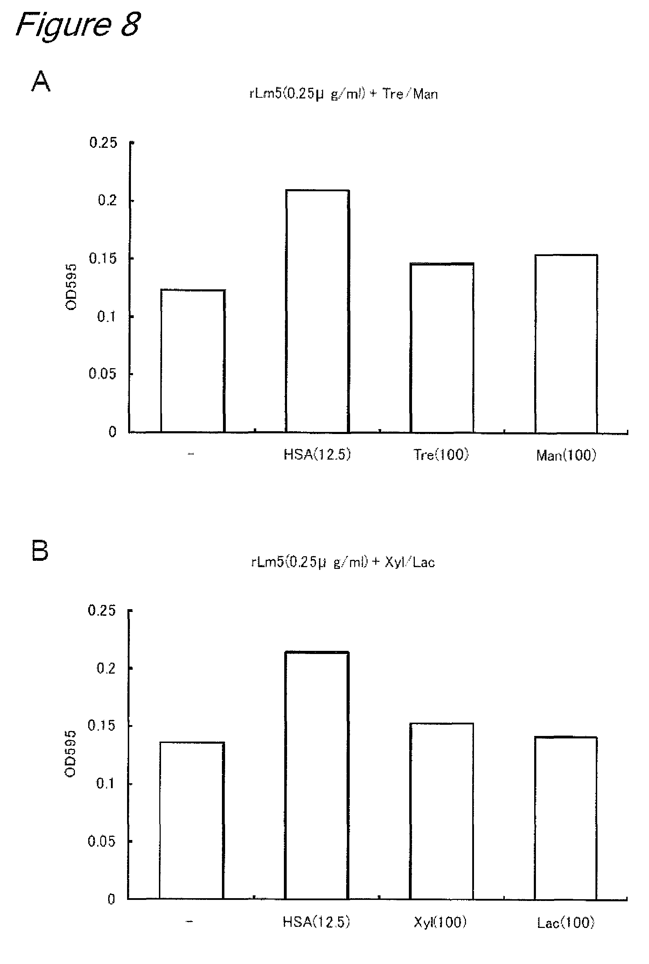

Figure 8 shows the result of an investigation on whether recombinant human laminin-5 (0.25 µg/ml) and various sugars affect the cell adhesion activity against BRL cells at a higher concentration. Figure 8A shows results for Tre and Man each at 100 µg/ml. Figure 8B shows results for Xyl and Lac at 100 µg/ml.

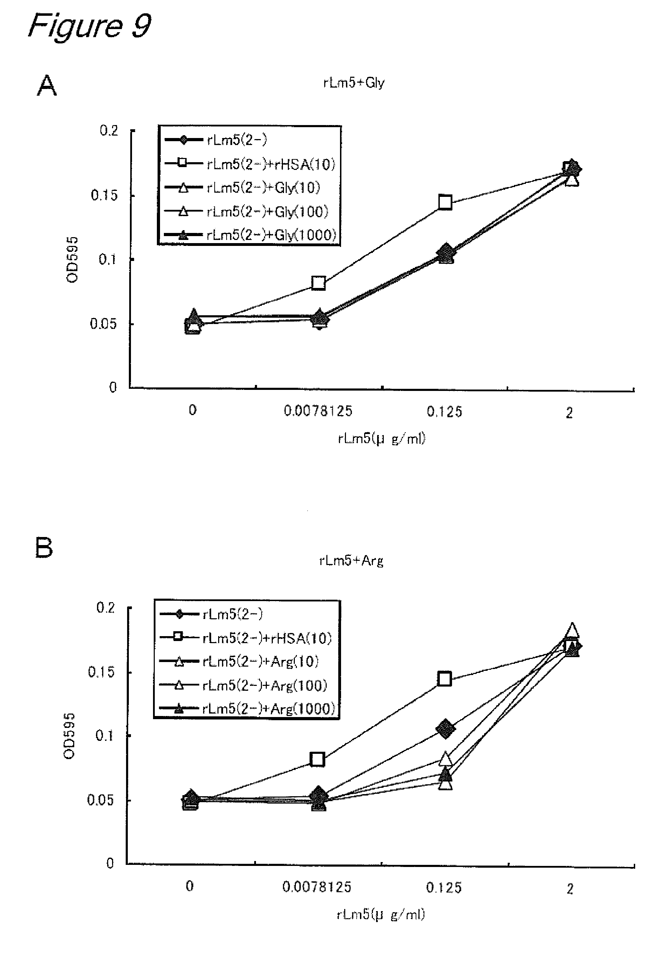

Figure 9 shows the result of an investigation on whether recombinant human laminin-5 (0.25µg/ml) and various amino acids are effective on the cell adhesion activity against BRL cells.
   Figure 9A
      Black diamond: just recombinant human laminin-5 (control)
      White square: recombinant human laminin-5 + HSA 10 µg/ml
      White triangle: recombinant human laminin-5 + glycine (Gly) 10 µg/ml
      Grey triangle: recombinant human laminin-5 + Gly 100 µg/ml
      Black triangle: recombinant human laminin-5 + Gly 1000 µg/ml
   Figure 9B
      Black diamond: just recombinant human laminin-5 (control)
      White square: recombinant human laminin-5 + HSA 10 µg/ml
      White triangle: recombinant human laminin-5 + arginine (Arg) 10 µg/ml
      Grey triangle: recombinant human laminin-5 + Arg 100 µg/ml
      Black triangle: recombinant human laminin-5 + Arg 1000 µg/ml
Figure 10 shows a synergetic effect of recombinant human laminin-5 on the cell adhesion activity when two types of proteins in blood are used. The proteins in blood on the x-axis were each used at the indicated concentration.
Figure 11 shows the result of an assessment to determine the order of treating the cell incubator. Recombinant human laminin-5 of concentrations provided on the x-axis (0-2 µg/ml) was used.
   Black diamond: just rLm5
   White square: the cell incubation plate was treated with rLm5 after it was treated with rHSA (rHSA→rLm5)
   White triangle: the cell incubation plate was treated with rHSA and rLm5 at the same time (rLm5 + rHSA)
   Cross: the cell incubation plate was treated with rHSA after it was treated with rLm5 (rLm5→rHSA)

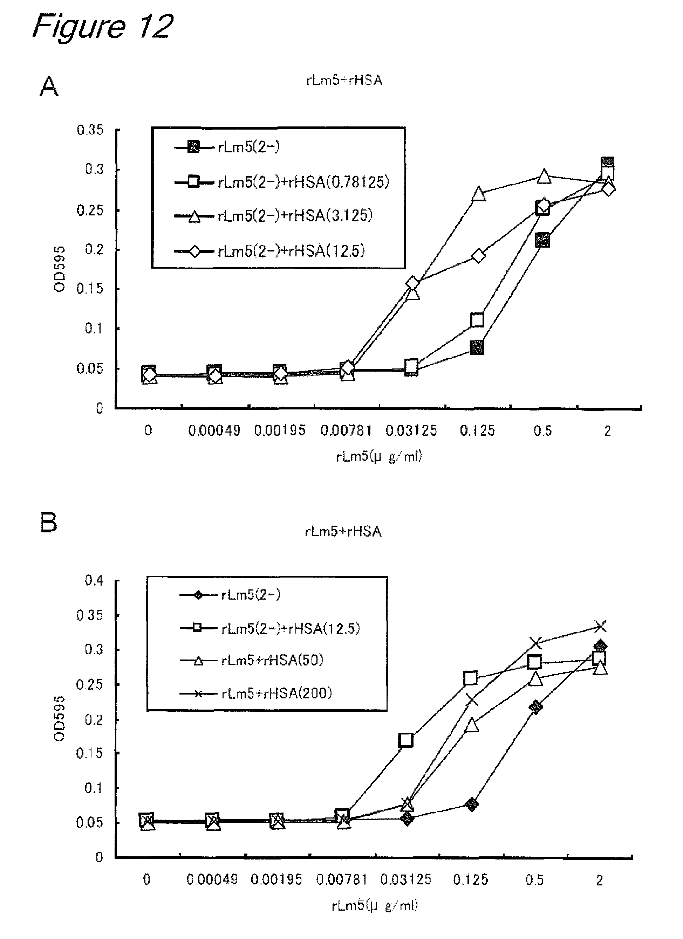

Figure 12 shows the result of an investigation for the optimal concentration of recombinant HSA (rHSA) to increase the cell adhesion activity of the recombinant human laminin-5 at concentrations provided on the x-axis (0-2 µg/ml) against HT1080 cells.
   Figure 12A
      Black diamond: just recombinant human laminin-5 (control)
      White square: recombinant human laminin-5 + rHSA 0.78125 µg/ml
      White triangle: recombinant human laminin-5 + rHSA 3.125 µg/ml
      Cross: recombinant human laminin-5 + rHSA 12.5 µg/ml
   Figure 12B
      Black diamond: just recombinant human laminin-5 (control)
      White square: recombinant human laminin-5 + rHSA 12.5 µg/ml
      White triangle: recombinant human laminin-5 + rHSA 50 µg/ml
      Cross: recombinant human laminin-5 + rHSA 200 µg/ml
Figure 13 shows the result of an investigation for the optimal concentration of recombinant HSA (rHSA) to increase the cell adhesion activity of the recombinant human laminin-5 at concentrations provided on the x-axis (0-2 µg/ml) against human mesenchymal stem cells (hMSC).
   Figure 13A
      Black diamond: just recombinant human laminin-5 (control)
      White square: recombinant human laminin-5 + rHSA 0.78125 µg/ml
      White triangle: recombinant human laminin-5 + rHSA 3.125 µg/ml
      Cross: recombinant human laminin-5 + rHSA 12.5 µg/ml
   Figure 13B
      Black diamond: just recombinant human laminin-5 (control)
      White square: recombinant human laminin-5 + rHSA 12.5 µg/ml
      White triangle: recombinant human laminin-5 + rHSA 50 µg/ml
      Cross: recombinant human laminin-5 + rHSA 200 µg/ml
Figure 14 shows the result of an investigation for the optimal concentration of recombinant HSA (rHSA) to increase the cell adhesion activity of the recombinant human laminin-5 at concentrations provided on the x-axis (0-16 µg/ml) against EB3 cells.
   Figure 14A
      Black diamond: just recombinant human laminin-5 (control)
      White square: recombinant human laminin-5 + rHSA 0.78125 µg/ml
      White triangle: recombinant human laminin-5 + rHSA 3.125 µg/ml
      Cross: recombinant human laminin-5 + rHSA 12.5 µg/ml
   Figure 14B
      Black diamond: just recombinant human laminin-5 (control)
      White square: recombinant human laminin-5 + rHSA 12.5 µg/ml
      White triangle: recombinant human laminin-5 + rHSA 50 µg/ml
      Cross: recombinant human laminin-5 + rHSA 200 µg/ml
Figure 15 consists of photographs showing the cell state of recombinant human laminin-5, at concentrations provided in the figure, after analyzing the adhesion of EB3 cells for cases with rHSA (12.5 µg/ml) added to it. The photographs on the left are results of not using rHSA (control), and those on the right are results of using rHSA.

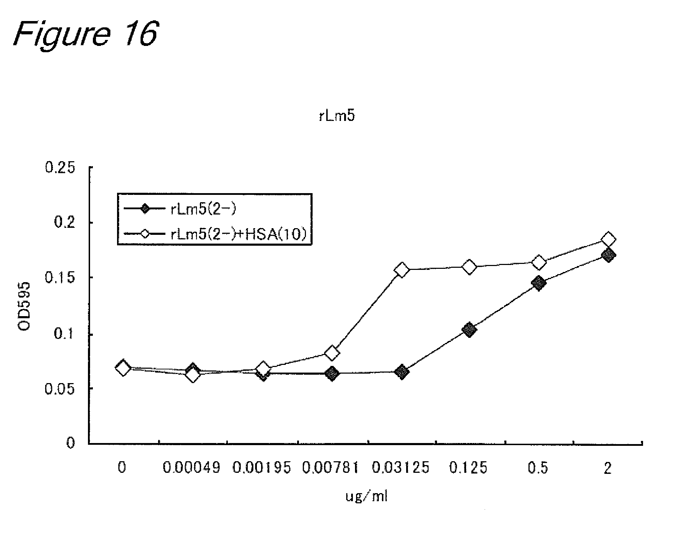

Figure 16 shows the result of an investigation on whether HSA exhibits elevating effects on the cell adhesion activity against laminin isoforms other than human laminin-5, as it does for laminin-5. Laminin-5 was used at concentrations provided on the x-axis (0-2 µg/ml).
   Black diamond: just recombinant human laminin-5 (control)
   White diamond: recombinant human laminin-5 + HSA 10 µg/ml
Figure 17 shows the result of an investigation on whether rHSA exhibits elevating effects on the cell adhesion activity against extracellular matrix proteins and isoforms other than human laminin-5, as it does for laminin-5. Vitronectin (Vn/SIGMA) was used as the other extracellular matrix protein, and laminin 2 (Lm2/Millipore) was used as the other laminin isoform. Laminin-5 (0-2 µg/ml) and vitronection (0-32 µg/ml), Lm2 (0-32 µg/ml) were each used at concentrations provided on the x-axis.
   Figure 17A
      Black diamond: just recombinant human laminin-5 (control)
      White diamond: recombinant human laminin-5 + rHSA 10 µg/ml
      Black circle: just Vn (control)
      White circle: Vn + rHSA 10 µg/ml
   Figure 17B
      Black diamond: just recombinant human laminin-5 (control)
      White diamond: recombinant human laminin-5 + rHSA 10 µg/ml
      Black square: just Lm2 (control)
      White square: Lm2 + rHSA 10 µg/ml
Figure 18 shows a result of an investigation on the activity elevating effects of rHSA (10 µg/ml) on the cell scattering activity of recombinant human laminin-5 at concentrations provided on the x-axis against BRL cells. The activity elevating effects of rHSA was observed to be especially high when the concentration of rLm5 was at 0.02 µg/ml.

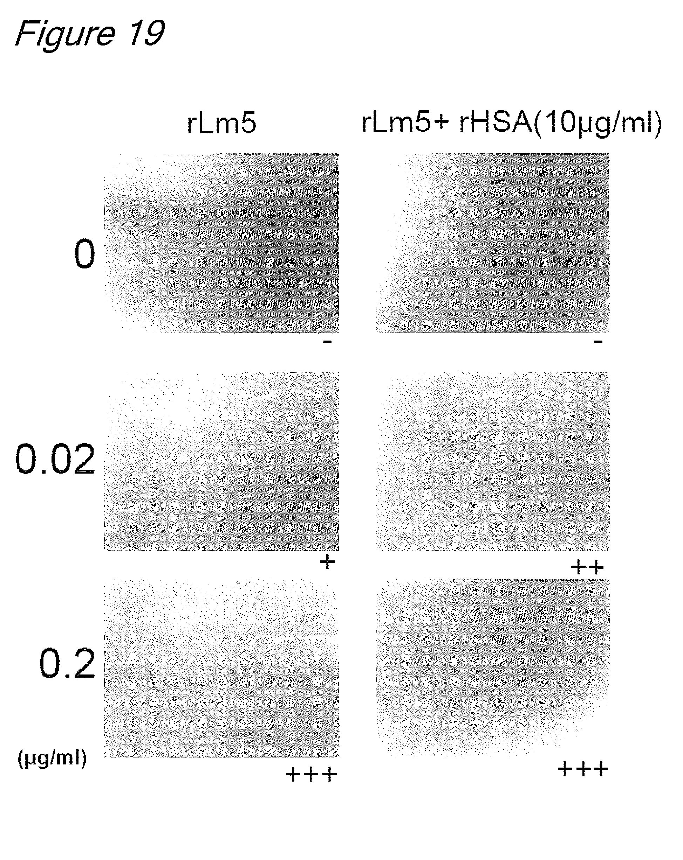

Figure 19 is photographs showing the result of an investigation on the activity elevating effects of rHSA (10 µg/ml) concerning the cell scattering activity of recombinant human laminin-5 at the given concentrations (0-0.2 µg/ml) against BRL cells.

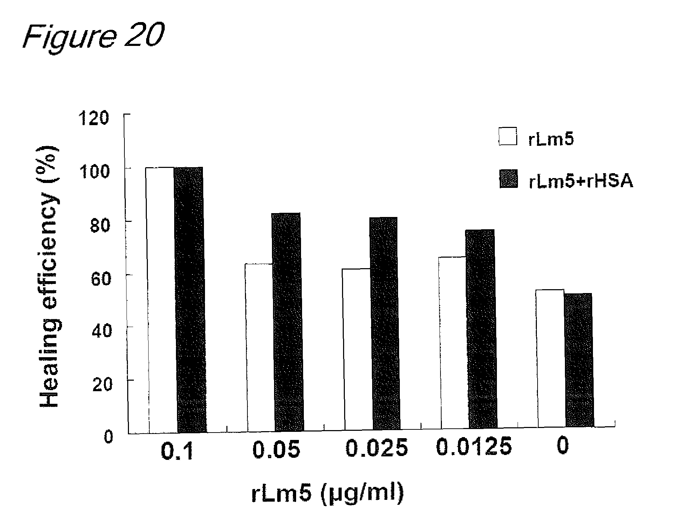

Figure 20 shows the result of an investigation on the activity elevating effects of rHSA (10 µg/ml) concerning the wound healing activity of recombinant human laminin-5 at concentrations provided on the x-axis (0-0.1 g/ml) against BRL cells.

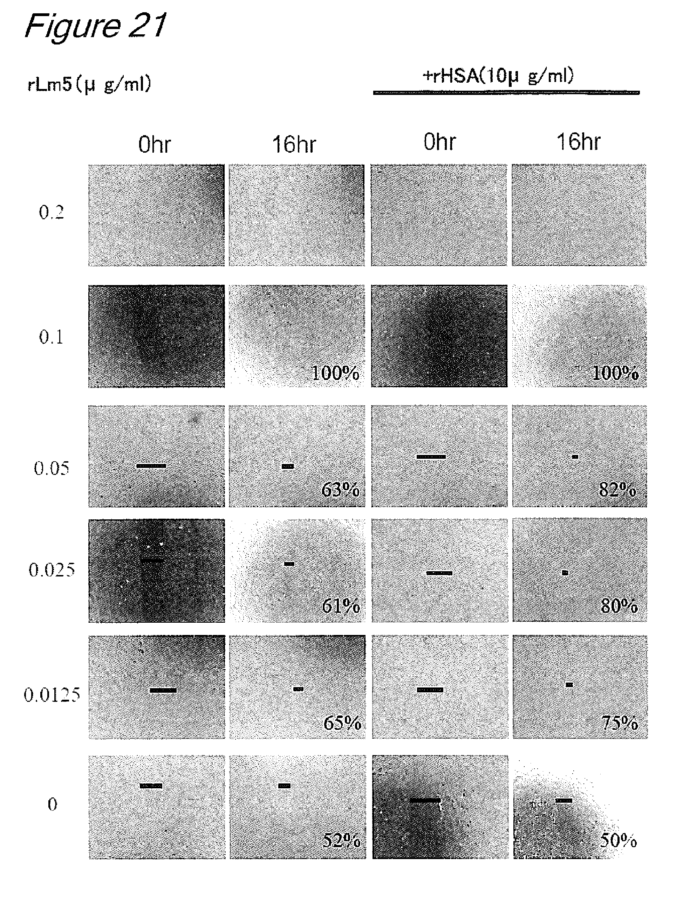

Figure 21 is photographs showing the result of an investigation on the activity elevating effects of rHSA (10 µg/ml) concerning the wound healing activity of recombinant human laminin-5 at concentrations (0-0.1 g/ml) against BRL cells.

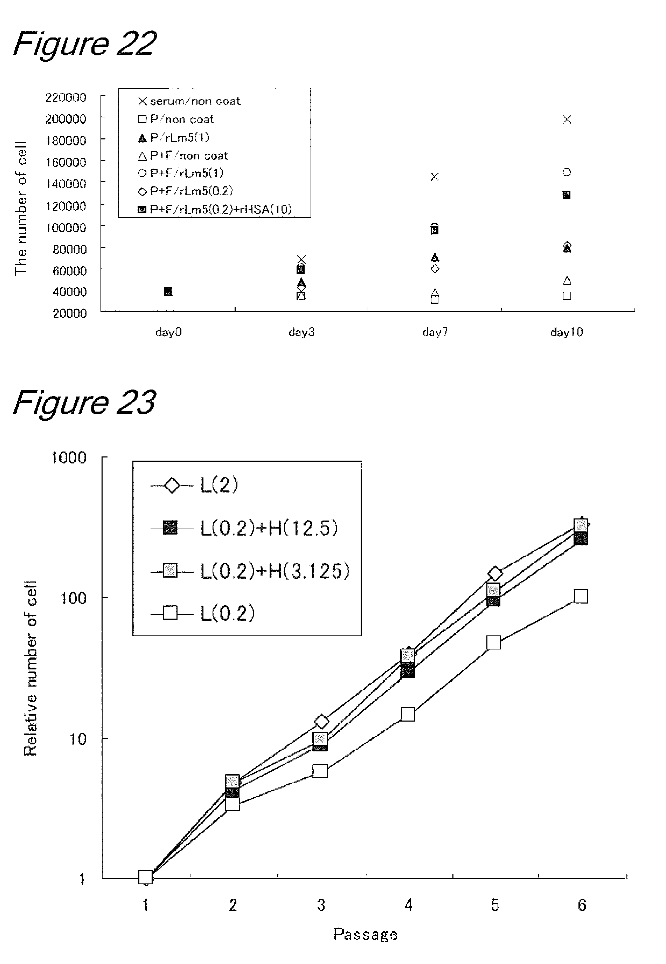

Figure 22 shows the result of an investigation on the activity elevating effects of rHSA (10 µg/ml) concerning the proliferation activity of recombinant human laminin-5 against hMBC cells.
   Cross: only serums without coating
   White square: Panexin is added (P)
   Black triangle: P/rLm5 1 µg/ml
   White triangle: P + F (bFGF)/no coating
   White circle: P + F/rLm5 1 µg/ml
   White diamond: P + F/rLm5 0.2 µg/ml
   Black square: P + F/rLm5 0.2 µg/ml + rHSA 10 µg/ml
Figure 23 shows the result of an investigation on the activity elevating effects of rHSA (10 µg/ml) concerning the proliferation activity of recombinant human laminin-5 against EB3cells.
   White diamond: Lm5 (2 µg/ml)
   Black square: Lm5 (0.2 µg/ml) + rHSA 12.5 µg/ml
   Grey square: Lm5 (0.2 µg/ml) + rHSA 3.125 µg/ml
   White square: Lm5 (0.2 µg/ml)

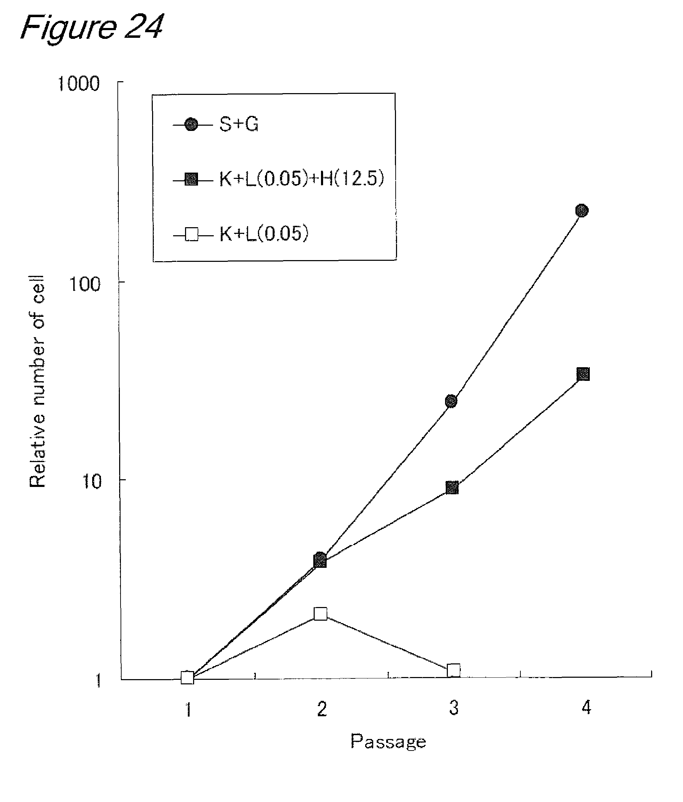

Figure 24 shows the result of an investigation on the activity elevating effects of rHSA concerning the proliferation activity of recombinant human laminin-5 against EB3cells for various cell supporting materials that are used.
   Black circle: proliferation medium (S) + bovine gelatin (G)
   Black square: KSR-GMEM (K) + Lm5 (L) (0.05 µg/ml) + rHSA (H) (12.5 µg/ml)
   White square: KSR-GMEM (K) + Lm5 (L) (0.05 µg/ml)

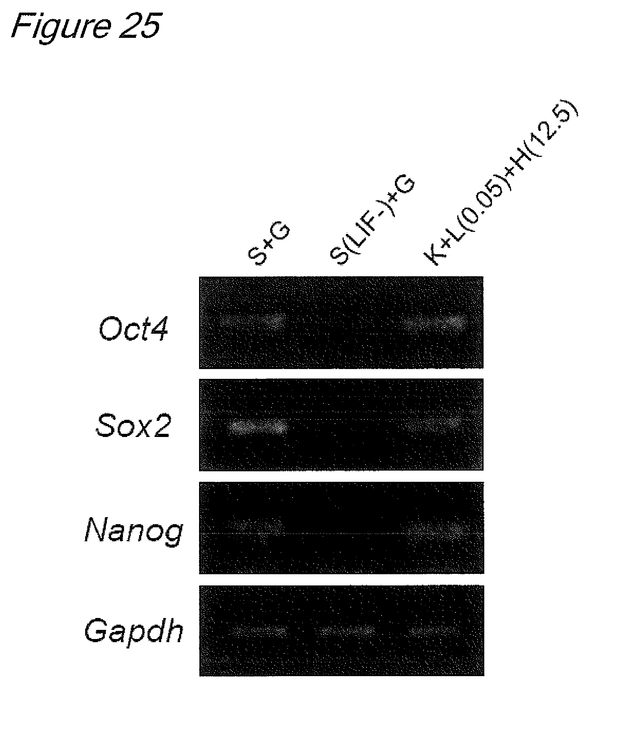

Figure 25 shows the result of an undifferentiated-state marker detection concerning S+G and K + L (0.05 µg/ml) + H (12.5 µg/ml), which both exhibited proliferation in Figure 24.

### DESCRIPTION OF EMBODIMENTS

The present invention relates to a method for culturing cells in a system containing laminin-5.

The method of the present invention is a method for culturing cells in a system containing laminin-5, characterized by a culture system comprising a polypeptide selected from a group consisting of: a protein in blood other than extracellular matrix proteins, which is, serum, serum albumin, prealbumin, immunoglobulin, α-globulin, β-globulin, α1-antitrypsin (α1-AT), heptoglobin (Hp), α2-macroglobulin (α2-M), α-fetoprotein (AFP), transferrin, retinol-binding protein (RBP) or adiponectin; gelatin; a protein belonging to a tumor necrosis factor (TNF) family; and peptone.

### Laminin-5

The method of the present invention is directed to the culture of pluripotent stem cells and its most remarkable feature lies in culturing the pluripotent stem cells in a system containing laminin-5.

The adhesion activity of Laminin-5 against many cell types is reported to be stronger than those of various extracellular matrix proteins including other laminin isoforms (J. Biochem. 116:862-869, 1994; J. Cell Biol. 125:205-214, 1994; Mol BiolCell. 16:881-890, 2005).

As shown in Table 1, laminin-5 is a laminin molecule composed of α3, β3 and γ2 chains, which plays a dominant role in binding epidermis and corium, and binds preferentially to integrin a3β1 in most cells and also binds to integrin α6β1 or α6β4 in some cells. In laminin-5, it has been elucidated that the α3G2A sequence (RERFNISTPAFRGCMKNLKKTS) in the α3 chain G2 domain and the KRD sequence in the G3 domain are the major binding sites for integrin.

It is also known that laminin-5, after being secreted as a trimer, receives limited proteolysis by protease to remove G4 and G5 domains located at the C-terminal of the α3 chain, and is thereby converted from 190 kDa (nontruncated) into 160 kDa (truncated). Laminin-5 isolated in a standard manner does not have G4 and G5 domains. Such α3 chain-truncated laminin-5 is known to have higher stimulating activities on cell adhesion, motility and neuranagenesis, when compared to non-truncated laminin-5 (J. Biol. Chem., 280 (2005): 14370-14377).

Laminin-5 in the present invention is not particularly limited, and may be either in a non-truncated form containingG4 and G5 domains or in a truncated form free from all or part of G4 and G5 domains.

Moreover, the laminin-5 protein may be either naturally occurring or modified to have one or more modified amino acid residues while maintaining its biological activities, particularly stimulating activity on cell adhesion. Moreover, the laminin-5 protein in the present invention may be of any origin and may be prepared in any manner, as long as it has the features described herein. Namely, the laminin-5 protein of the present invention may be naturally occurring, expressed from recombinant DNA by genetic engineering procedures, or chemically synthesized.

The laminin-5 protein may be of any origin, preferably of human origin. In a case where human pluripotent stem cells are cultured in order to obtain materials for regenerative medicine, etc., it is preferred to use laminin-5 of human origin for the sake of avoiding the use of materials derived from other animals.

SEQ ID NOs: 1 to 6 in the Sequence Listing herein show the nucleotide and amino acid sequences of human laminin-5 α3 chain, the nucleotide and amino acid sequences of human laminin-5 β3 chain and the nucleotide and amino acid sequences of human laminin-5 γ2 chain, respectively. The laminin-5 protein to be used in the present invention is preferably a protein composed of the following subunits: an α3 chain having the amino acid sequence of SEQ ID NO: 2 or an amino acid sequence comprising deletion, addition or substitution of one or more amino acids in the sequence of SEQ ID NO:2 (amino acid residues 1-1713) (J. Biol. Chem. 269:22779-22787, 1994), a β3 chain having the amino acid sequence of SEQ ID NO: 4 or an amino acid sequence comprising deletion, addition or substitution of one or more amino acids in the sequence of SEQ ID NO: 4 (amino acid residues 1-1170) (1. Biol. Chem. 269:11073-11080, 1994), and a γ2 chain having the amino acid sequence of SEQ ID NO: 6 or an amino acid sequence comprising deletion, addition or substitution of one or more amino acids in the sequence of SEQ ID NO: 6 (amino acid residues 1-1193) (J. Cell. Biol. 119:679-693, 1992).

Globular domains (G1 to G5 domains) in the α3 chain correspond to amino acid residues 794-970, 971-1139, 1140-1353, 1354-1529 and 1530-1713, respectively, in SEQ ID NO: 1.

Each chain of laminin-5 may have an amino acid sequence comprising deletion, addition or substitution of one or more amino acid residues in the amino acid sequence shown in the corresponding SEQ ID NO. Such proteins having amino acid sequences homologous to naturally occurring proteins can also be used in the present invention. The number of modifiable amino acids is not particularly limited in the amino acid sequences of α3, β3 andγ2 chains, but it is preferably 1 to 300 amino acid residues, 1 to 200 amino acid residues, 1 to 150 amino acid residues, 1 to 120 amino acid residues, 1 to 100 amino acid residues, 1 to 80 amino acid residues, 1 to 50 amino acid residues, 1 to 30 amino acid residues, 1 to 20 amino acid residues, 1 to 15 amino acid residues, 1 to 10 amino acid residues, or 1 to 5 amino acid residues. More preferably, it is a number of amino acid residues modifiable by known site-directed mutagenesis, for example, 1 to 10 amino acid residues, or 1 to 5 amino acid residues.

It is well known in the art that conservative substitution of amino acids can be used to obtain proteins or polypeptides maintaining their original functions. Such substitution includes replacement of an amino acid with another residue having similar physical and chemical properties, as exemplified by replacement of one fatty acid residue (Ile, Val, Leu or Ala) with another, or replacement between basic residues Lys and Arg, between acidic residues Glu and Asp, between amide residues Gln and Asn, between hydroxyl residues Ser and Tyr, or between aromatic residues Phe and Tyr.

Laminin-5 to be used in the present invention may also be a protein sharing at least 80%, 85%, 90%, 95%, 98% or 99% identity with the amino acid sequences shown in SEQ ID NOs: 2, 4 and 6 and having the ability to stimulate cell adhesion activity.

Identity is calculated as follows: the number of identical residues is divided by the total number of residues in a corresponding known sequence or a domain therein, and then multiplied by 100. Computer programs available for use in the determination of sequence identity using standard parameters include, for example, Gapped BLAST PSI-BLAST (Nucleic Acids Res. 25:3389-3402, 1997), BLAST (J. Mol. Biol. 215:403-410, 1990), and Smith-Waterman (J. Mol. Biol. 147:195-197, 1981). In these programs, default settings are preferably used, but these settings may be modified, if desired.

The laminin-5 protein in the present invention may be of any origin and may be prepared in any manner, as long as it has the features described herein. Namely, the laminin-5 protein of the present invention may be a naturally occurring laminin-5 protein as found in or purified from the supernatant of human or animal cells secreting laminin-5. However, laminin-5 can be effectively produced as a recombinant protein by expressing each subunit using recombinant DNA technology known in the art. It is particularly preferred to obtain laminin-5 as a human recombinant protein, for the sake of avoiding unwanted factors derived from other animals.

For this purpose, primers may be designed based on a DNA sequence comprising nucleic acid residues 1-5139 in SEQ ID NO: 1 (encoding the laminin-5 α3 chain) and nucleotide sequences of nucleic acid residues 121-3630 in SEQID NO: 3 (encoding the β3 chain) and nucleic acid residues 118-3696 in SEQ ID NO: 5 (encoding the γ2 chain), and an appropriate cDNA library may be used as a template in polymerase chain reaction (PCR) to amplify desired sequences. Such PCR procedures are well known in the art and can be found, e.g., in "PCR Protocols, A Guide to Methods and Applications," Academic Press, Michael, et al., 1990.

DNA encoding a gene of each chain of laminin-5 may be integrated into an appropriate vector and then introduced into either eukaryotic or prokaryotic cells by using an expression vector that allows expression in each host, whereby the respective chains are expressed to obtain a desired protein. Host cells which can be used to express laminin-5 are not limited in any way and include prokaryotic host cells such as E. coli and Bacillus subtilis, as well aseukaryotic hosts such as yeast, fungi, insect cells and mammalian cells.

Vector constructed to express laminin-5 can be introduced into the above host cells by transformation, transfection, conjugation, protoplast fusion, electroporation, particle gun technique, calcium phosphate precipitation, direct microinjection or other techniques. The cells containing the vector may be grown in an appropriate medium to produce a laminin-5 protein to be used in the present invention, which may then be purified from the cells or medium to obtain the laminin-5 protein. Purification may be accomplished, for example, by size exclusion chromatography, HPLC, ion exchange chromatography, immunoaffinity chromatography, etc.

Laminin-5 is described in detail in JP 2001-172196 A, which is incorporated herein by reference.

Laminin is structured as a heterotrimer molecule composed of α, β and γ chains linked to each other via disulfide linkages, which takes a characteristic cross-structure. Each chain is composed of two or more domains, and domains I and II form a triple helix. Before the filing of the present application, at least 15 isoforms of laminin molecules have been identified from different combinations of 5 types of α chains (α1 to α5), 3 types of β chains (β1 to β3) and 3 types of γ chains (γ1 to γ3), and it is suggested that there are actually several times that number of isoforms. Laminin 1, which is a typical laminin, is a hetero trimer molecule composed of α1, β1, γ1, and laminin-5 used in the present application is composed of α3, β3, γ2. For example, when the homology of the polypeptide chains of laminin-1 and laminin-5 are analyzed using software such as Genetyx, the homology of α1 and α3 is 42%, that of β1 and β3 is 41%, and that of γ1 and γ2 is 54%. Although laminin-1 and laminin-5 are both laminins, they are presumed to show different characteristics, because they respectively have compositions, (α1, β1, γ1) and (α3, β3, γ2), consisting of α, β, γ chains, wherein each of the three chains are encoded by a completely different gene than that of the other laminin, and further, even in the same α chain, α1 and α3, encoded by two different genes has a homology of merely 42%.

As shown in Example 3, no activity elevating effect was exhibited for vitronectin, which is another extracellular matrix, and laminin-2, which is another laminin isoform, as shown in Example 3. (Figure 17) As shown above, the activity elevating effect is not a phenomenon common to all extracellular matrix proteins, and further, it is not a phenomenon common to any isoform of laminin.

### Polypeptide

The present invention is characterized by the increase in the various activities of Lm5 in cell culture in combination with a specific polypeptide in a cell culturing system containing Lm5.

Polypeptide is selected from a group consisting of: a protein in blood other than extracellular matrix proteins, which is, serum, serum albumin, prealbumin, immunoglobulin, α-globulin, β-globulin, α1-antitrypsin (α1-AT), heptoglobin (Hp), α2-macroglobulin (α2-M), α-fetoprotein (AFP), transferrin, retinol-binding protein (RBP) or adiponectin; gelatin; a protein belonging to a tumor necrosis factor (TNF) family; and peptone.

### 1) Protein in blood

The present invention preferably uses protein in blood, more preferably protein in blood other than the extracellular matrix protein, with laminin-5 protein.

The protein in blood is preferably selected from a protein in blood other than extracellular matrix proteins, which is, serum, serum albumin, prealbumin, immunoglobulin, α-globulin, β-globulin, α1-antitrypsin (α1-AT), heptoglobin (Hp), α2-macroglobulin (α2-M), α-fetoprotein (AFP), transferrin, retinol-binding protein (RBP) or adiponectin. These are all proteins in blood other than the extracellular matrix protein.

An "extracellular matrix" is a substance filling the extracellular space. At the same time, it acts as a bone structure (e.g. cartilage or bone of an animal), a foothold for cell adhesion (e.g. basement membranes or fibronectin), and a retainer and a provider of cell growth factor (e.g. a cell growth factor binding to heparan sulfate, i.e. FGF). It may be said that many cells constituting multicellular organisms live hidden in a bed or a nest of the extracellular matrix. Essential ingredients in the extracellular matrix of vertebrates including human are glycoprotein such as collagen, proteoglycan, fibronectin or laminin (some are cell adhesion molecules). An "extracellular matrix protein" is a protein constituting the above extracellular matrix.

The "protein in blood other than the extracellular matrix protein" in the present invention is those proteins in blood that are not extracellular matrix proteins related to cell adhesion and other activities. These are all proteins known in the art that a person skilled in the art can obtain as necessary.

The "protein in blood other than extracellular matrix protein" is not limited, but it is preferably human serum albumin (HSA/ obtainable, for example, from Nacalai), recombinant human serum albumin (rHSA/ obtainable, for example, from SIGMA), or bovine serum albumin (BSA/ obtainable, for example, from SIGMA).

The "protein in blood other than extracellular matrix protein" may be immunoglobulin. Immunoglobulin is well known by a person skilled in the art, and includes IgG, IgA, IgM, IgD, IgE. A human immunoglobulin (IgG/e.g. obtainable from Oriental Yeast Co., Ltd.) may be used, for example.

### 2) Gelatin

Gelatin is extracted from collagen, which is the main ingredient of connective tissues, such as the skin, the bones, and the tendon of an animal, by heating the collagen. The main ingredient of gelatin is protein.

### 3) Protein in the tumor necrosis factor (TNF) family

"Tumor Necrosis Factor (TNF)" is a type of cytokine, consisting of the following three types by narrow definition: TNF-α, TNF-β (lymphotoxin (LT)-α) and LT-β. The "protein in the TNF family" includes at least 19 types of molecules including receptor activating factor NFkB ligand (RANKL), Fas ligand, CD40 ligand.

Receptor activating factor NFkB ligand (RANKL) can be preferably used as an example of "protein in the TNF family" of the present invention.

### 4) Peptone

A "peptone" is protein digested by protease. Protein is digested in the stomach by pepsin to become peptone, and then peptone is further digested to amino acid by the pancreatic juice secreted from the pancreas and the intestinal juice secreted from jejunum.

Peptone is often added to the culture, since it is a suitable nutrition source of microorganisms. The peptone acting as a nutrition source in the culture is protein hydrolysized to amino acid and peptide with low molecular weight, commonly using an enzymolysis product (e.g. protease such as pancreatin derived from the pancreas of a swine) of milk protein (milk casein).

Any peptone can be used, but it is preferable to use a plant derived peptone. It is selected, for example, from a group consisting of a cotton seed-derived peptone, a soy bean-derived peptone, a wheat-derived peptone and a pea-derived peptone.

The effect of the present invention to increase the activity of laminin-5 was not obtained from a simple amino acid as shown in Example 1. Accordingly, the "peptide" of the present invention does not include a peptone digested to a simple amino acid.

### Cells

The types and origins of cells to be cultured in the method of the present invention are not particularly limited.

Cells are preferably selected from a group consisting of pluripotent stem cells, tissue stem cells, somatic cells, germ cells and sacroma cells. Any pluripotent stem cells can be used, but they are preferably selected from embryonic stem cells, induced pluripotent stem cells, embryonic germ cells or germline stem cells. Tissue stem cells are preferably selected from mesenchymal stem cells, hepatic stem cells, pancreatic stem cells, neural stem cells, skin stem cells or hematopoietic stem cells. The somatic cells are preferably selected from hepatic cells, pancreatic cells, muscle cells, osteocytes, osteoblasts, osteoclasts, cartilage cells, fat cells, skin cells, fibroblasts, pancreatic cells, kidney cells, pneumocytes or blood cells, which are lymphocites, red blood cells, white blood cells, monocytes, macrophage or megakaryocytes.

Species of organisms acting as the origin of the cells are not particularly limited either. Cells are preferably derived from mammals such as mice, rats, humans, monkeys, pigs, dogs, sheep and goats and birds such as chickens. More preferably, they are derived from the species selected from a group consisting of mice, rats and humans.

As used herein, the term "pluripotent stem cells" is intended to collectively refer to stem cells capable of differentiating into cells of any tissue type (pluripotency). Although ES cells (EB3 cells) are used for study in the Examples below, pluripotent stem cells that can be used in the method of the present invention include not only embryonic stem cells, but also all pluripotent stem cells derived from, e.g., cells of adult mammalian organs or tissues, bone marrow cells, blood cells, and embryonic or fetal cells, as long as their characteristics are similar to those of embryonic stem cells. Characteristics similar to embryonic stem cells in the present context can be defined as a cytobiological features specific to embryonic stem cells, which are a gene expression specific to embryonic stem cells and differentiation capability to all germ layers, such as endoderm, mesoderm, and ectoderm.

Specific examples of cells that can be proliferated by the method of the present invention include, but are not limited to, embryonic stem cells (ES cells), induced pluripotent stem cells (iPS cells), embryonic germ cells (EG cells), germline stem cells (GS cells) and so on. It should be noted that pluripotent stem cells preferred in the present invention are ES cells and iPS cells. An iPS cell is especially preferable for reasons including that it poses no ethical problem. Any known pluripotent stem cell can be used. An example is the pluripotent stem cells described in International Publication WO 2009/123349 (PCT/JP 2009/057041).

"Tissue stem cells" are stem cells that can be differentiated to various cell species (pluripotency), although only cell lines of specific tissues are differentiable. For example, hematopoietic stem cells in the bone marrow form blood and neural stem cells differentiate into nerve cells. A variety of other cells are included, such as hepatic stem cells to create the liver, and skin stem cells to create skin tissue.

"Somatic cells" are cells, other than germ cells, constituting the multicellular organisms. These cells are not inherited to the next generation in a sexual reproduction. The term in the present specification represents various cells other than the "pluripotent stem cells" and "tissue stem cells".

### System comprising laminin-5

The present invention cultures cells in a system containing laminin-5. The terms "system containing laminin-5" in the present invention means inclution of laminin-5 in the cell culture system in one form or another, without any limitation to the embodiment.

An embodiment of using a culture vessel treated by laminin-5, especially coated by laminin-5, is preferable for culturing cells in a system containing laminin-5 in the present invention.

A "cell culture vessel" is not particularly limited in the present invention, and a vessel sterilized to prevent contamination by germs, made of any material and any shape suitable for culturing cells can be used. Examples of such culture vessel include a culture dish, a culture flask, a culture schale, a culture plate with 96 wells, 48 wells, 12 wells, 6 wells or 4 wells and so on, and a culture bottle, which are commonly used in the present field of art, without being limited thereby.

The present invention is characterized by using laminin-5 and polypeptide in cell culture. Preferably, the surface of the cell culture vessel is treated by immobilizing (coating) laminin-5 and polypeptide. Treating the culture vessel by immobilizing laminin-5 on its surface is a treatment technique known in the art, and a person skilled in the art may adopt any culture vessel depending on the purpose of the present invention to treat the vessel with laminin-5 and polypeptide and use the treated vessel to cultured cells by the method of the present invention.

The amount of laminin-5 used in the treatment of the cell culture vessel is not particularly limited. A good result is obtained when the vessel is treated with a solution containing preferably 0.01 µg/ml or more, preferably 0.1-15 µg/ml, and more preferably 0.1 µg/ml-2 µg/ml of laminin-5.

A treatment of the culture vessel by laminin-5 in an embodiment of the present invention may comprise drying or other treatments after depositing laminin-5 on the inner surface of the culture vessel. A culture medium commonly used for cell culture, such as GMEM (GIBCO) and DMEM, may be placed in a culture vessel treated with laminin-5, and pluripotent stem cells are added to that culture medium. Then, cells are cultured under appropriate culture conditions known in the art, such as, at 37°C, in 5% carbon dioxide air layer, without being limited thereby.

It is preferable in the present invention to treat (coat) the cell culture vessel with laminin-5 and polypeptide. The order of treating a cell culture vessel is not particularly limited, but preferably, it is treated with polypeptide before it is treated with laminin, or it is treated with polypeptide and laminin at the same time.

Accordingly, the present invention also provides compositions for coating the cell culture vessel or a cell culture vessel coating agent comprising the aforementioned polypeptide. The composition or agent of the present invention may contain laminin-5 in combination with the polypeptide. The present invention further provides a kit comprising the cell culture medium containing the above mentioned polypeptide. The present invention also provides a kit comprising the cell culture medium containing polypeptide and laminin-5 aforementioned. The kit of the present invention may also include a precoated culture dish, a precoated culture plate or the like other than the cell culture medium.

The composition, the agent or the kit of the present invention can be used in a method for culturing cells in a system containing laminin-5.

### Amount of Polypeptide to be used

The amount of Polypeptide to be used is not particularly limited in the present invention. A person skilled in the art can appropriately select an appropriate amount depending on elements such as the type of polypeptide to be used.

Polypeptide is preferably used at a concentration of 1 µg/ml to 200 µg/ml, without being limited thereby. Example 1 showed that a concentration of 3.125 µg/ml to 12.5 µg/ml is especially preferable when protein in blood, such as HSA, BSA, HS or IgG is used. Further, when a cotton seed derived peptone was used, the cell activity elevating effect against laminin-5 was exhibited at higher concentrations, preferably 15.6 µg/ml to 1000 µg/ml.

### Combined Use of Polypeptide

In a preferable embodiment of the present invention, two or more types of polypeptide are included in the cell culturing system. When rHSA and IgG were combined for use as polypeptide in Example 1, an adhesive activity as strong as that of using 10 µg/ml of rHSA was obtained, even though each was used at a low concentration (0.25 µg/ml) that would not provide any large effect alone. Accordingly, it is considered that the combined use of two or more types of polypeptides provides a synergistic effect, not an additive effect.

### Effects of the Present Invention

Various activities of laminin-5 protein induced in the cell culture of the present invention increase when laminin-5 is combined for use with polypeptide. The effects of laminin-5 include a cell adhesion activity, a cell scattering activity, a wound healing activity, a proliferation stimulating activity, an activity for maintaining undifferentiated-state and an activity for maintaining pluripotency, without being limited thereby.

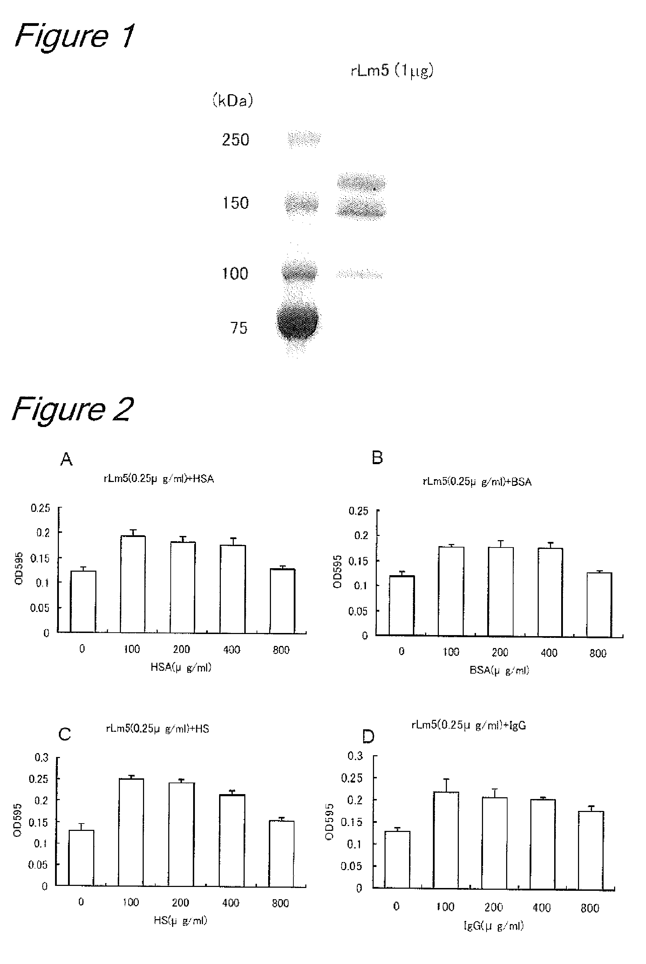

A "cell adhesion activity" is an effect of inducing cell adhesion. In Figure 2, Figure 3 and Figure 4, a combined use of HSA with rLm5 (0.125 µg/ml) produced an adhesion activity equivalent to that of rLm5 (2 µg/ml). Likewise, in Figure 13, a combined used of HSA with rLm5 (0.5 µg/ml) produced an adhesion activity equivalent to that of rLm5 (2 µg/ml). Hence, the use of polypeptide increases the cell adhesion activity of the present invention to preferably 1.2 times or more, more preferably 4 times or more, most preferably 8 times or more the activity of an invention not using polypeptide.

A "cell scattering activity" is an effect of scattering cells. The use of polypeptide increases the cell scattering activity of the present invention to preferably twice the activity of an invention not using polypeptide.

A "wound healing activity" is an effect of healing wounds. In other words, it is an activity brought about by the coating of, for example, laminin-5 on a physically injured section abandoned by the cells, resulting in the migration of surrounding cells to the coated section. The wound healing effect can be determined by assessing the healing ratio through measuring the wound width after specific periods from the time the subject has been wounded (e.g. after 16 hours). The combined use of polypeptide (rHSA) with human laminin-5 in Example 5 increased the wound healing percentage from 60% to 80%.

A "cell proliferation activity" is an effect of advancing cell proliferation. The effect of cell proliferation can be determined by measuring the number of cells after a specific length of time from the start of cell culture.

An "activity for mainting undifferentiated-state" is the effect of maintaining the undifferentiated state when the cells to be cultured are undifferentiated cells, such as, pluripotent stem cells or tissue stem cells. Culturing the cells with laiminin-5 keeps the cells from being differentiated and maintains the undifferentiated state. The undifferentiated state is also maintained when laminin-5 and polypeptide are combined for use (Example 7, Figure 25). Sox2, Nanog, Oct4 and other undifferentiated-state markers can be measured to confirm whether the undifferentiated state is maintained during culture.

An "activity mainting pluripotency" is the act of maintaining pluripotency when the cells to be cultured have pluripotency, for example, when they are pluripotent stem cells. Pluripotency is also maintained when laminin-5 and polypeptide are combined for use in the present invention.

### EXAMPLES

The present invention will now be described in more detail below on the basis of the following examples, which are not intended to limit the scope of the invention.

### Example 1: Preparation of recombinant human laminin-5 (rLm5)

In this example, a recombinant human laminin-5 protein was prepared in a known manner.

From human fetal kidney cell line HEK293 modified to carry cDNAs for α3 chain (SEQ ID NO: 1), β3 chain (SEQ ID NO: 3) and γ2 chain (SEQ ID NO: 5) (Lm5-HEK293), the serum-free supernatant was collected and centrifuged at 4°C at 3000 rpm for 5 minutes. The human fetal kidney cell line HEK293 was obtained as described in J. Biochem.132:607-612 (2002). The supernatant was then applied to Heparin sepharose CL-6B (GE healthcare) and eluted. The rLm5-containing fractions were passed through an antibody column, in which mouse anti-Lm-α3 (anti-laminin α3) monoclonal antibody (BG5) was covalently bonded to Protein A sepharose CL-6B (GE healthcare), and then eluted. It should be noted that monoclonal antibody BG5 is an antibody prepared by the inventors of the present invention using an N terminal fragment of the laminin α3B chain as an antigen according to known procedures for monoclonal antibody preparation.

Purified rLm5 (1 µg) was denaturated under reducing conditions and then subjected to SDS polyacrylamide gel electrophoresis on a 5-20% gel to confirm the size and purity of α3, β3 and γ2 chains. As a result, bands of 160 kDa, 135 kDa and 105 kDa were detected, respectively. Figure 1 shows a photograph of SDS polyacrylamide gel electrophoresis obtained for purified rLm5. When analyzed with a CS-Analyzer, purified rLm5 was found to have a purity of about 98%. rLm5 thus prepared was used in the following examples.

### Example 2: Cell adhesion assay

This example shows the result of an adhesion assay when rLm5 is added to various cells and when additives are added in addition to rLm5.

Four types of cells, specifically, rat hepatic cell line (BRL), mouse ES cell line (EB3), human sarcoma cell line (HT1080), human mesenchymal stem cell (hMSC), were used. BRL was offered by Yokohama City University, Graduate School of Nanobioscience, Department of Genome System Science. EB3 was offered by Osaka University, Graduate School of Medicine, Frontier Biosciences G6, Course on Molecular Treatment, Field of Stem Cell Regulation. HT1080 was obtained from Riken BioResource Center (RCB1956). hMSC was obtained from Lonza Corporation.

Different types of cells were cultured and proliferated in the following culture media: BRL in DMEM/F12 with 1.0% fetal bovine serum (FBS) added to it; EB3 in GMEM (GIBCO) with 10% FBS, 0.1 mM of non-essential amino acid (Gibco), 1 mM of sodium pyruvate (Gibco), 1000 U/ml of ESGRO (Millipore), and 10⁻⁴ M of 2-mercaptoethanol (WAKO); HT1080 in MEM (SIGMA) with 10% FBS added to it; and hMSC in MSCGM (LONZA). A serum-free medium, that is, media wherein serum has been removed, were used to perform the adhesion assay.

A 96-well plate (Corning) was treated with a concentration-controlled rLm5 at 37°C for 2 hours or at 4°C overnight, then the treated surface was washed with PBS(-), and was subjected to 1 hour of blocking treatment at 37°C. The rLm5 used in the treatment was mixed with human serum albumin (HSA/Nacalai), recombinant human serum albumin (rHSA/SIGMA), human serum (HS/OYC), bovine serum albumin (BSA/SIGMA), human immunoglobulin (IgG/OYC), bovine gelatin (Gl/SIGMA), recombinant human Receptor Activator of NF-κB Ligand (sRANKL/OYC), cotton seed-derived peptone (Pep/DMV), glycine (Gly/Nacalai), arginine (Arg/Nacalai), trehalose (Tre/SIGMA), xylose (Xyl/Wako), mannose (Man/Wako), lactose (Lac/Wako) as necessary. The cells were washed in a serum-free medium, i.e., media to which no serum was added, then, they were seeded in the well at 20000 cells/well, and cultured for 1 hour at 37°C, under an air layer of 5% CO₂ and 95% air. However, EB3 was seeded in the well at 30000 cells/well. After culturing completed, the plates were lightly shaken by the Vortex mixer to let cells with weak adhesion float off to be removed by Percoll (GE healthcare) treatment. The adhered cells were fixed by 25% glutaraldehyde (Nacalai), dyed with 2.5% crystal violet (Nacalai), and measured with OD595 to assess the adhesion activity of rLm5 at various conditions.

Figures 2-11 show the result of the adhesion assay using BRL; Figure 12 shows the result of the adhesion assay using HT1080; Figure 13 shows the result of the adhesion assay using hMSC; and Figures 14, 15 show the result of the adhesion assay using EB3.

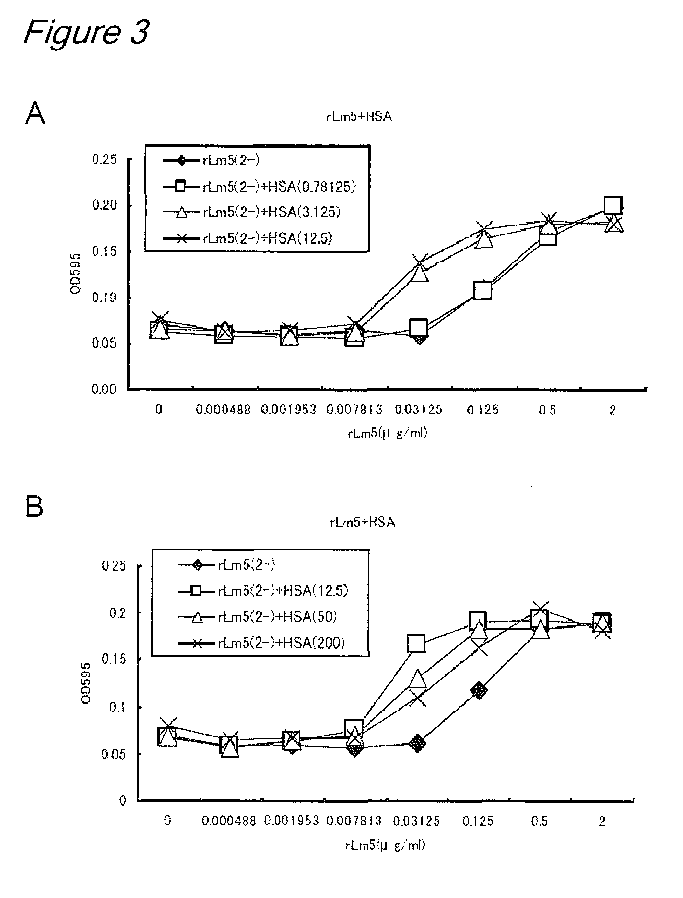

In a treatment with 0.25 µg/ml of rLm5, the adhesion activity was higher when HSA, BSA, HS, IgG were combined for use than when they were not. This result showed that protein in blood is effective in increasing the adhesion activity of rLm5 (Figure 2). HSA (0-200 µg/ml) were combined for use to determine the optimum concentration of HSA. The result was that the optimum concentration is in the range of 3.125-12.5 µg/ml (Figure 3). Further, 0-200 µg/ml of rHSA was combined for use to identify the substance showing the activity elevating effect. The result showed that rHSA has an optimum concentration in the range of 3.125-12.5 µg/ml, like HSA. It was thus concluded that the activity elevating effect is not exhibited by substances such as impurities in the naturally occurring protein (Figure 4).

Then, Gl, sRANKL, Pep were used to determine whether proteins other than proteins in blood, such as HSA, BSA, HS, IgG, have the same effects. The result was that the effects differed slightly by the substance, but they commonly had adhesion activity elevating effects like HSA (Figure 5). Although the activity elevating effect of Pep was minute, an evaluation performed at a wider range of concentrations (0-1000 µg/ml) showed that Pep exhibits a strong activity elevating effect at high concentrations (Figure 6). From the above observations, it was concluded that polypeptide or peptide would exhibit an activity elevating effect.

Sugars were assessed next. A combined use of Xyl, Man, Lac or Tre with 0.25 µg/ml of rLm5 did not produce an activity elevating effect like that of the combined use of polypeptide or peptide (Figure 7). A combined use at a higher concentration (100 µg/ml) did not present a strong activity elevating effect like HSA (Figure 8).

Amino acids were assessed next. They were similar to sugars in that a combined use of 0-1000 µg/ml of Gly or Arg with rLm5 did not provide a strong activity elevating effect like that of rHSA (Figure 9). The activity decreased for Arg.

The above results show that activity elevating effects are not exhibited by low molecules such as sugars and amino acids.

A combined use of multiple proteins was assessed next. The adhesion activity was low when either 0.5 µg/ml of rHSA alone or 0.25 µg/ml of IgG alone was combined for use with 0.125 µg/ml of rLm5 relative to when 10 µg/ml of rHSA was combined for use with the same rLm5. However, when 0.5 µg/ml of rHSA and 0.25 µg/ml of IgG were both combined for use with rLm5, the adhesion activity was equivalent to that of using 10 µg/ml of rHSA (Figure 10). The result showed that a combination of multiple types of proteins increases the adhesion activity of rLm5.

The combination method was subsequently assessed using 10 µg/ml of rHSA. The following cases were compared: a case of treating with rHSA before treating with rLm5 (rHSA→rLm5); the conventional method of treating with rHSA and rLM5 at the same time (rLm5+rHSA); and treating with rLm5 before treating with rHSA (rLm5→rHSA). The result showed that the use of rHSA in the preliminary treatment or together with rLm5 provides adhesion activity elevating effects, but the use of rHSA in the later treatment does not provide adhesion activity elevating effects (Figure 11).

Next, HT1080, hMSC, EB3 were used to assess whether activity elevating effects like those in BRL are exhibited in cells other than BRL. The assessment was conducted with 0-200 µg/ml of rHSA used in combination with rLm5. The result was that activity elevating effects were seen in all cells, although there were some differences in the activity elevating effects according to the cell type. Further, optimum concentration was exhibited is in the range of 3.125-12.5 µg/ml like BRL (Figures 12-14). The morphology of EB3 cells after adhesion assay is shown in Figure 15.

The results showed that performing treatments by combining polypeptide or peptide with rLm5 will raise the adhesion activity of rLm5 in various cells. Further, the optimum concentration of polypeptide combined for use is 3.125-12.5 µg/ml, and the polypeptide or peptide to be combined does not have to be used at the same time as rLm5, but it can be used in advance and still increase the rLm5 activity sufficiently.

### Example 3: Assessment of activity elevating effects of other extracellular matrix proteins or laminin isoforms

This example shows the result of an assessment by an adhesion assay using rat hepatic cell line (BRL) to consider whether an activity elevating effect similar to that of rLm5 is exhibited in other extracellular matrix proteins or laminin isoforms, such as human vitronectin (Vn/SIGMA) and human laminin 2 (Lm2/Millipore). The assay was conducted in accordance with the method described in Example 2.

Figure 16 and Figure 17 respectively show the result of an adhesion assay in which 10 µg/ml of HSA and 10 µg/ml of rHSA are combined. Activity elevating effects from combination with HSA or rHSA, as exhibited for rLm5, were not exhibited at all for Vn and Lm2. Such result indicates that the activity elevating effect is not a phenomenon occurring to any extracellular matrix protein, moreover, the phenomenon is not common to all isoforms of laminin.

### Example 4: Cell scattering assay using BRL cells

This example shows the result of a cell scattering assay when rLm5 is added to rat hepatic cell line (BRL) cells and when additives are added in addition to rLm5. BRL was offered by Yokohama City University, Graduate School of Nanobioscience, Department of Genome System Science.

BRL cells were cultured and proliferated in DMEM/F12 with 10% fetal bovine serum (FBS) added to it. However, the cell scattering assay was conducted using DMEM/F12 culture with 1% FBS added to it.

A 24-well plate (Nunc) was treated with a concentration-controlled rLm5 at 4°C overnight, then the treated surface was washed with PBS(-), and t was subjected to 1 hour of blocking treatment in a 1% BSA (SIGMA) solution at 37°C. The rLm5 used in the treatment was mixed with rHSA as necessary. After washing the rLm5-treated surface with PBS(-), the cells were washed in a 1% FBS culture, then they were seeded in the well at 7000 cells/well and subsequently cultured for 40 hours at 37°C, under an air layer of 5% CO₂ and 95% air. After culturing completed, the plates were lightly shaken by the Vortex mixer to let cells with weak adhesion float off to be removed by Percoll treatment. The adhered cells were fixed by 25% glutaraldehyde. Photographs of 3 random fields were taken to count the number of single cell.

The result of the cell scattering assay is shown in Figures 18 and 19. A significant increase in the cell scattering activity was recognized when rHSA was added to Lm5 of a low concentration (0.02 µg/ml).

The result indicates that an activity elevating effect of combining rHSA and other polypeptides for use is recognized not only for the cell adhesion activity, but also for the cell scattering activity, which is already reported as an activity of rLm5.

### Example 5: Wound Healing Assay using BRL Cells

This example shows the result of a wound healing assay when rLm5 is added to rat hepatic cell line (BRL) cells and when additives are added in addition to rLm5. BRL was offered by Yokohama City University, Graduate School of Nanobioscience, Department of Genome System Science.

BRL cells were cultured and proliferated in DMEM/F12 with 10% fetal bovine serum (FBS) added to it. However, the wound healing assay was conducted using medium with serum-free mediummedium, having no serum therein, added to the medium.

Cells were seeded to a 10% FBS culture at 160000 cells/well in a 24-well plate (Nunc), then they were cultured for 3 hours at 37°C, under an air layer of 5% CO₂ and 95% air. After culturing completed, a blue chip was used to put an injury of a specific width on the adhesion cell group, then the cells were washed twice in a serum-free medium, i.e., media wherein serum has been removed. The rLm5 that was concentration controlled in the serum-free medium was used to treat the wells at 37°C for 1 hour. Treatment with rLm5 was conducted by mixing rHSA with rLm5 as necessary. After treatment with rLm5 completed, the treated surface was washed twice with serum-free medium, created by removing serum, and serum-free medium was added. The result was observed through a microscope, and the area around the wound was photographed. Then, the result was cultured for 16 hours at 37°C, under an air layer of 5% CO₂ and 95% air in a serum-free medium, and the same area was photographed (Figure 21). The healing of the wound was assessed by measuring the width of the wound at the beginning and that at 16 hours therefrom to obtain the wound healing ratio.

Figure 20 shows the obtained wound healing ratio. A significant increase in the wound healing activity was exhibited when rHSA was added to Lm5.

The result indicates that an activity elevating effect of combining rHSA and other polypeptides for use is recognized not only for the cell adhesion activity and the cell scattering activity, but also for the wound healing activity previously reported as an activity of rLm5.

### Example 6: Proliferation Assay using human mesenchymal stem cells

This example shows the result of a proliferation assay using hMSC when different cell supporting materials are used.

Themedia used in the proliferation assay were medium with 5% Panexin (PAN-biotech) added to it instead of 10% FBS (P) and medium with 5% Panexin and 1 ng/ml of bFGF(Wako Pure Chemical) added to it (P+F). A maintenance medium (serum) comprising 10% FBS was also used for comparison. hMSC in each medium were seeded at 38400 cells/well in a 6 well plate (NUNC) treated with concentration-controlled extracellular matrix protein. They were cultured at 37°C, under an air layer of 5% CO₂ and 95% air for 10 days, and the cells were collected on the 3^{rd} day, the 7^{th} day and the 10^{th} day from the beginning of culturing by enzyme treatment to count the number of cells using a hemacytometer.

The cell supporting material was prepared to form a mixture with 1 mg/ml of rLm5, 0.2 µg/ml of rLm5, 0.2 µg/ml of rLm5 and 10 µg/ml of rHSA added to it.

Figure 22 shows the result of an investigation on the effect of hMSC on proliferation under different culture conditions. A significant increase in cell proliferation was seen when rHSA was added to 0.2 µg/ml of rLm5.

The above result indicates that an activity elevating effect of combining rHSA and other polypeptides for use is recognized not only for the cell adhesion activity, the cell scattering activity and the wound healing activity, but also for the proliferation stimulating activity for mesenchymal stem cells which is already reported as an activity of rLm5. Hence, it is presumed that rHSA and other polypeptides can increase all activities of rLm5 when they are used in combination with rLm5.

### Example 7: Proliferation Assay using EB3

This example shows result of a proliferation assay using EB3 when rLm5 is used as a cell supporting material.

The maintenance medium used for EB3 was medium (KSR-GMEM) with 10% Knockout™ Serum Replacement additives (KSR) (Invitrogen) instead of 10% FBS of the proliferation culture of Example 2. EB3 was seeded at 43000 cells/well to a 12 well plate (NUNC) treated with concentration-controlled extracellular matrix proteins of different types. They were cultured at 37°C, under an air layer of 5% CO₂ and 95% air for 2 days, then they were collected by enzyme treatment to count the number of cells using a hemacytometer.

EB3 was seeded again at 43000 cells/well to a 12 well plated treated with a concentration-controlled rLm5. The proliferation effects of rLm5 to EB3 at different conditions were compared by repeating the above process. The following variations of rLm5 were prepared: 2 µg/ml (L(2)), 0.2 µg/ml (L(0.2)), 0.2 µg/ml of rLm5 with 3.125 µg/ml of rHSA added to it (L(0.2)+H(3.125)), 0.2 µg/ml of rLm5 with 12.5 µg/ml of rHSA added to it (L(0.2)+H(12.5)).

Figure 23 shows the result of a theoretical calculation showing by what factor EB3 will proliferate after 5 passages under the different culture conditions. According to the result shown in Figure 23, the cell proliferation of the experiment using 0.2 µg/ml of rLm5 with rHSA added to it was about 3 times as much as the cell proliferation of only 0.2µg/ml of rLm5, and equivalent to that of 2 µg/ml of rLm5.

The result shows that the effect of combination with rHSA is exhibited in the proliferation of mouse ES cells as well.

### Example 8: Proliferation assay using EB3 and the detection of an undifferentiated state markers

This example shows the result of a proliferation assay using EB3 and the detection result for an undifferentiated-state marker when different cell supporting materials are used.

The Example was performed with the same number of cells seeded in the proliferation assay of EB3 and the same passage interval as Example 7. The proliferation medium (S) used in Example 2 and KSR-GMEM (K) were used as the medium for the proliferation assay. The extracellular matrix proteins used for treating a 12 well plate were 1 mg/ml of Gl, 0.05 µg/ml of rLm5 (L0.05), and 0.05 µg/ml of rLm5 with 12.5 µg/ml of HSA added to it (L0.05 + H12.5).

Figure 24 shows the result of a theoretical calculation showing by what factor EB3 will proliferate after 3 passages under the given culture conditions. The result in Figure 24 shows that proliferation terminates midway in an experiment using only 0.05 µg/ml of rLm5, but a substantial proliferation is exhibited in the experiment using 0.05 µg/ml of rLm5 with rHSA added to it.

Next, S+G, K+L (0.05)+H (12.5) which exhibited proliferation were subjected to RT-PCR to detect undifferentiated-state markers, specifically to detect gene expression of Oct4, Sox2, Nanog, which are known in the art as undifferentiated-state markers of mouse ES cells. Note that under the condition of S+G, an experiment area maintained for the same period without LIF, which is known as the undifferentiated-state maintenance factor of mouse ES cells, was set as the negative control area (S(LIF-)+Gl).

TRIZOL (Invitrogen) was used to extract all RNAs from EB3 that has been cultured for 3 passages. After the extraction completed, reverse transcription was performed using ThermoScript RT-PCR System (Invitrogen) for cDNA synthesis. The primer of Table 2 was used to perform PCR using the synthesized cDNA as the mould.

**[Table 2]**

| RT-PCR primer |
|---|
| Nanog |
| 5'-AAGCAGAAGATGCGGACTGT-3' (SEQ ID NO:7) |
| 5'-ACCACTGGTTTTTCTGCCAC-3' (SEQ ID NO:8) |

| Oct4 |
|---|
| 5'-TCTTTCCACCAGGCCCCCGGCTC-3' (SEQ ID NO:9) |
| S'-TGCGGGCGGACATGGGGAGATCC-3' (SEQ ID NO:10) |

| Sox2 |
|---|
| 5'-TAGAGCTAGACTCCGGGCGATGA-3' (SEQ ID NO:11) |
| 5'-TTGCCTTAAACAAGACCACGAAA-3' (SEQ ID NO:12) |

| Gapdh |
|---|
| 5'-CACCATGGAGAAGGCCGGGG-3' (SEQ ID NO:13) |
| 5'-GACGGACACATTGGGGGTAG-3' (SEQ ID NO:14) |

The degeneration reaction of the genes were performed at 94°C, for 30 seconds, the annealing reaction was performed for 30 seconds, and the extension reaction was performed at 72°C, for 20 seconds. The annealing reaction was performed at 61°C for Oct4, and at 54°C for Sox2, and Nanog.

Figure 25 shows the detection result of the undifferentiated-state markers using RT-PCR. The result of Figure 25 shows that 3 undifferentiated-state markers are expressed for K + L(0.05) + H(12.5) similar to S + Gl. This result suggests that EB3 maintains its undifferentiated state even when rHSA is combined for use with rLm5.

The result shows that the conbination of rLm5 and rHSA not only has advantageous effects in proliferation, but it also maintains the proper undifferentiated-state of mouse ES cells for the retained mouse ES cells..

### SEQUENCE LISTING FREE TEXT

<SEQ ID NO: 1>
   SEQ ID NO: 1 shows the nucleotide sequence of human laminin α3 chain.
<SEQ ID NO: 2>
   SEQ ID NO: 2 shows the amino acid sequence of human laminin α3 chain.
<SEQ ID NO: 3>
   SEQ ID NO: 3 shows the nucleotide sequence of human laminin β3 chain.
<SEQ ID NO: 4>
   SEQ ID NO: 4 shows the amino acid sequence of human laminin β3 chain.
<SEQ ID NO: 5>
   SEQ ID NO: 5 shows the nucleotide sequence of human laminin γ2 chain.
<SEQ ID NO: 6>
   SEQ ID NO: 6 shows the amino acid sequence of human laminin γ2 chain.
<SEQ ID NOs: 7 to 14>
   SEQ ID NOs: 7 to 14 show the nucleotide sequences of RT-PCR primers used for undifferentiated-state marker detection in EB3 cells.

## Claims

1. A method for culturing cells in a system containing laminin-5 **characterized by** a culture system comprising a polypeptide selected from a group consisting of: a protein in blood other than extracellular matrix proteins, which is, serum, serum albumin, prealbumin, immunoglobulin, α-globulin, β-globulin, α1-antitrypsin (α1-AT), heptoglobin (Hp), α2-macroglobulin (α2-M), α-fetoprotein (AFP), transferrin, retinol-binding protein (RBP) or adiponectin; gelatin; a protein belonging to a tumor necrosis factor (TNF) family; and peptone.

2. The method according to Claim 1, wherein the protein belonging to the tumor necrosis factor (TNF) family is a receptor activator NFₖB ligand (RANKL).

3. The method according to Claim 1, wherein peptone is selected from a group consisting of a cotton seed derived peptone, a soy bean derived peptone, a wheat derived peptone and a pea derived peptone.

4. The method according to any one of Claims 1 to 3, wherein a cell culture vessel is treated with laminin-5 after it is treated with polypeptide, or the vessel is treated with polypeptide and laminin at the same time.

5. The method according to any one of Claims 1 to 4, wherein an activity of laminin-5 against the cells, selected from a group consisting of a cell adhesion activity, a cell scattering activity, a wound healing activity, a proliferation stimulating activity, an activity for mainting undifferentiated-state and an activity for mainting pluripotency, is increased.

6. The method according to any one of Claims 1 to 5, wherein the cells are selected from a group consisting of pluripotent stem cells, tissue stem cells, somatic cells, germ cells and sacroma cells.

7. The method according to Claim 6, wherein the pluripotent stem cells are selected from embryonic stem cells, induced pluripotent stem cells, embryonic germ cells or germline stem cells;
the tissue stem cells are selected from mesenchymal stem cells, hepatic stem cells, pancreatic stem cells, neural stem cells, skin stem cells or hematopoietic stem cells; or
the somatic cells are selected from hepatic cells, pancreatic cells, muscle cells, osteocytes, osteoblasts, osteoclasts, cartilage cells, fat cells, skin cells, fibroblasts, pancreatic cells, kidney cells, pneumocytes or blood cells, which are lymphocites, red blood cells, white blood cells, monocytes, macrophage or megakaryocytes.

8. The method according to any one of Claims 1 to 5, wherein the cells are derived from a species selected from a group consisting of a mouse, a rat or a human.

9. The method according to any one of Claims 1 to 8, wherein the polypeptide is used at a concentration of 1 µg/ml to 200 µg/ml.

10. The method according to any one of Claims 1 to 8, wherein the polypeptide is used at a concentration of 3.125 µg/ml to 12.5 µg/ml.

11. The method according to any one of Claims 1 to 8, wherein the cell culture system comprises two or more types of polypeptides.

12. A composition to be used in a method for culturing cells in a system comprising laminin-5, wherein the composition comprises a polypeptide selected from a group consisting of: a protein in blood other than extracellular matrix proteins, which is, serum, serum albumin, prealbumin, immunoglobulin, α-globulin, β-globulin, α1-antitrypsin (α1-AT), heptoglobin (Hp), α2-macroglobulin (α2-M), α-fetoprotein (AFP), transferrin, retinol-binding protein (RBP) or adiponectin; gelatin; a protein belonging to a tumor necrosis factor (TNF) family; and peptone.

13. The composition according to Claim 12, which further comprises laminin-5.

14. A kit for use in a method for culturing cells in a system comprising laminin-5, wherein the system comprises a polypeptide selected from a group consisting of: a protein in blood other than extracellular matrix proteins, which is, serum, serum albumin, prealbumin, immunoglobulin, α-globulin, β-globulin, α1-antitrypsin (α1-AT), heptoglobin (Hp), α2-macroglobulin (α2-M), α-fetoprotein (AFP), transferrin, retinol-binding protein (RBP) or adiponectin; gelatin; a protein belonging to a tumor necrosis factor (TNF) family; and peptone.

15. The kit according to Claim 14 further comprising laminin-5.
